(19) Europäisches Patentamt
European Patent Office
Office européen des brevets

(11) **EP 4 269 436 A1**

(12) **EUROPEAN PATENT APPLICATION**
published in accordance with Art. 153(4) EPC

(43) Date of publication:
**01.11.2023 Bulletin 2023/44**

(21) Application number: **21909573.4**

(22) Date of filing: **24.12.2021**

(51) International Patent Classification (IPC):
**C07K 16/28** (2006.01)     **A61K 39/395** (2006.01)
**A61P 35/00** (2006.01)     **A61P 35/02** (2006.01)

(52) Cooperative Patent Classification (CPC):
**A61K 39/395; A61P 35/00; A61P 35/02;
C07K 16/28**

(86) International application number:
**PCT/CN2021/141253**

(87) International publication number:
**WO 2022/135578 (30.06.2022 Gazette 2022/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.12.2020  CN 202011566537**

(71) Applicant: **Innovent Biologics (Suzhou) Co., Ltd.
Suzhou, Jiangsu 215123 (CN)**

(72) Inventors:
  • **JING, Hua
    Suzhou, Jiangsu 215123 (CN)**
  • **CHEN, Bingliang
    Suzhou, Jiangsu 215123 (CN)**
  • **LI, Li
    Suzhou, Jiangsu 215123 (CN)**

(74) Representative: **Sagittarius IP
Marlow International
Parkway
Marlow, SL7 1YL (GB)**

Remarks:
The complete document including Reference
Table(s) and the Sequence Listing(s) can be
downloaded from the EPO website

(54) **CLAUDIN18.2 CHIMERIC ANTIGEN RECEPTOR AND USE THEREOF**

(57)     The present invention generally relates to Claudin18.2 chimeric antigen receptors, T cells engineered to express Claudin18.2 chimeric antigen receptors (CARs), and uses for treating diseases associated with expression of Claudin18.2.

**Description**

The present invention generally relates to Claudin18.2 chimeric antigen receptors, T cells engineered to express Claudin18.2 chimeric antigen receptors (CARs), and uses for treating diseases associated with expression of Claudin18.2.

**BACKGROUND**

Claudins are a family of proteins and are important components that constitute tight junctions between cells. They establish an intercellular barrier that controls the intercellular flow of molecules, and thus can control the flow of the molecules between cells. Claudins family proteins have a tetraspanin region, both N-terminus and C-terminus of which are included in the cytoplasm. Different Claudins are expressed on different tissues and their functional changes are associated with the development of cancer in various tissues. For example, Claudin-1 is expressed in colon cancer and has prognostic value, Claudin-18 is expressed in gastric cancer, and Claudin-10 is expressed in hepatocellular carcinoma. Claudins, as cell membrane surface proteins, are useful targets of various therapeutic strategies.

Isoform 2 of Claudin-18 (Claudin18.2 or CLDN18.2) is a highly selective cell lineage marker. Its expression in normal tissues is strictly confined to differentiated epithelial cells of the gastric mucosa and its expression is absent from the gastric stem cell zone. CLDN18.2 is expressed in a considerable portion of primary gastric cancers, and its expression level is retained in gastric metastatic cancer tissue. Expression of CLDN18.2 has also been found in pancreatic cancer in addition to gastric cancer, and it is an ideal target molecule for the treatment of these cancers (Singh, P., Toom, S. & Huang, Y. Anti-CLDN18.2 antibody as new targeted therapy for advanced gastric cancer. J Hematol Oncol 10, 105 (2017). https://doi.org/10.1186/s13045-017-0473-4). Due to the large unmet clinical need for tumor therapy, the development of drugs targeting Claudin18.2 is necessary. Monoclonal antibodies against the Claudin18.2 target have entered the clinical phase II studies, and preliminary results show that the Claudin18.2 monoclonal antibody can significantly prolong the overall survival of patients with advanced gastric cancer through ADCC effect. The Claudin18.2 monoclonal antibody has the action mechanism of mobilizing NK cells to kill tumor cells. Claudin18.2/CD3 diabodies can kill a tumor by mobilizing endogenous T cells. However, the efficacy of both the above two techniques is limited by the ability of the patient's autoimmune cells to be activated to perform the function of killing tumor cells. T-cell therapy against this target is currently in its early stages. There is a need in the art to develop a T cell therapy, such as CART therapy, with higher tumor killing activity.

**SUMMARY**

In some aspects, the present invention relates to a chimeric antigen receptor (CAR) that binds to CLDN18.2, and a T cell comprising same.

In some aspects, the present invention also relates to a nucleic acid encoding the chimeric antigen receptor, and an expression vector, virus and T cell comprising same. The present invention also relates to the use of T cells comprising the chimeric antigen receptor for treating a tumor.

In some aspects, the present invention relates to the following specific embodiments:

1. An isolated chimeric antigen receptor comprising: an extracellular binding region, a transmembrane region, and an intracellular signaling region comprising a co-stimulatory domain, which are sequentially linked, wherein the extracellular binding region binds to CLDN18.2 and comprises a combination of HCDR1, HCDR2, HCDR3, LCDR1, LCDR2, and LCDR3 as follows:

| | SEQ ID NO | | | | | | | | | | |
|---|---|---|---|---|---|---|---|---|---|---|---|
| HCDR1 | 1 | 13 | 21 | 21 | 29 | 29 | 38 | 46 | 54 | 61 | 68 |
| HCDR2 | 2 | 14 | 22 | 88 | 30 | 30 | 39 | 47 | 55 | 62 | 69 |
| HCDR3 | 3 | 15 | 23 | 23 | 31 | 31 | 40 | 48 | 56 | 63 | 70 |
| LCDR1 | 6 | 6 | 6 | 6 | 33 | 89 | 42 | 50 | 50 | 50 | 72 |
| LCDR2 | 7 | 17 | 25 | 25 | 34 | 34 | 17 | 17 | 17 | 17 | 73 |
| LCDR3 | 8 | 18 | 26 | 26 | 35 | 35 | 43 | 51 | 58 | 65 | 74 |

2. The chimeric antigen receptor according to embodiment 1 wherein the extracellular binding region comprises a VH and a VL comprising or consisting of amino acid sequences shown below, respectively:

SEQ ID NOs: 4 and 9;

SEQ ID NOs: 16 and 19;

SEQ ID NOs: 24 and 27;

SEQ ID NOs: 86 and 27;

SEQ ID NOs: 32 and 36;

SEQ ID NOs: 32 and 87;

SEQ ID NOs: 41 and 44;

SEQ ID NOs: 49 and 52;

SEQ ID NOs: 57 and 59;

SEQ ID NOs: 64 and 66; or

SEQ ID NOs: 71 and 75.

3. The chimeric antigen receptor according to embodiment 1 or 2, wherein the extracellular binding region binding to CLDN18.2 is an antibody or an antigen binding fragment thereof, e.g., an scFv.

4. The chimeric antigen receptor according to embodiment 3, wherein the scFv comprises or consists of an amino acid sequence selected from SEQ ID NOs: 11, 20, 28, 37, 45, 53, 60, 67, and 76, or an amino acid sequence having at least 90%, 91%, 92%, 93%, 94%, 95%, 96%, 97%, 98%, or 99% identity to the amino acid sequence.

5. The chimeric antigen receptor according to any one of embodiments 1-4, wherein the transmembrane region is a CD8 (e.g., CD8α) or CD28 transmembrane region, preferably, the transmembrane region comprises or consists of a sequence of SEQ ID NO: 80 or 86, an amino acid sequence having at least 1, 2, or 3 modifications (e.g., substitutions) but not more than 20, 10, or 5 modifications (e.g., substitutions, e.g., conservative substitutions) relative to the amino acid sequence of SEQ ID NO: 80 or 86, or a sequence having at least 90% or 95% identity to the amino acid sequence of SEQ ID NO: 80 or 86; and optionally the transmembrane region is linked to the extracellular binding region via a hinge, and preferably, a hinge region comprises or consists of an amino acid sequence of SEQ ID NO: 79, or a sequence having at least 90%, 95%, 96%, or 97% identity to the amino acid sequence of SEQ ID NO: 79.

6. The chimeric antigen receptor according to any one of embodiments 1-5, wherein the intracellular signaling region comprises a CD3ζ signaling domain (e.g., comprising or consisting of a sequence set forth in SEQ ID NO: 82 or an amino acid sequence having at least 90%, 91%, 92%, 93%, 94%, 95%, 96%, 97%, 98%, or 99% identity thereto) and a co-stimulatory domain, preferably, the co-stimulatory domain is a functional signaling domain obtained from a protein selected from the group consisting of CD28 and 4-1BB (CD137), and preferably, the co-stimulatory domain comprises or consists of: an amino acid sequence having at least 1, 2 or 3 modifications (e.g., substitutions) but not more than 20, 10 or 5 modifications (e.g., substitutions, e.g., conservative substitutions) relative to an amino acid sequence of SEQ ID NO: 81 or 87, or a sequence having 90%, 91%, 92%, 93%, 94%, 95%, 96%, or 97% identity to the amino acid sequence of SEQ ID NO: 81 or 87.

7. The chimeric antigen receptor according to embodiment 6, wherein the co-stimulatory domain is located before the CD3ζ signaling domain.

8. The chimeric antigen receptor according to any one of embodiments 1-7, wherein the chimeric antigen receptor further comprises a signal peptide, e.g., a CD8 signal peptide, e.g., comprising or consisting of an amino acid sequence of SEQ ID NO: 78, and preferably, the signal peptide is located at the N-terminus of the extracellular binding region binding to CLDN18.2.

9. The chimeric antigen receptor according to any one of embodiments 1-8, wherein the chimeric antigen receptor further comprises a reporter gene, e.g., EGFP, e.g., comprising a sequence set forth in SEQ ID NO: 84 or an amino acid sequence having at least 90%, 91%, 92%, 93%, 94%, 95%, 96%, 97%, 98% or 99% identity thereto, and preferably, the CAR molecule further comprises a self-cleaving peptide linked to the reporter gene, e.g., T2A, facilitating cleavage of the reporter gene from the CAR molecule, and e.g., comprising or consisting of an amino acid sequence of SEQ ID NO: 83.

10. The chimeric antigen receptor according to any one of embodiments 1-9, wherein the chimeric antigen receptor sequentially comprises the signal peptide, the extracellular binding region binding to CLDN18.2, the hinge region, the transmembrane region, the intracellular signaling region comprising the co-stimulatory domain and the signaling domain, and optionally the self-cleaving peptide and the reporter gene.

11. A nucleic acid encoding the chimeric antigen receptor according to any one of embodiments 1-10.

12. An expression vector comprising the nucleic acid according to embodiment 11.

13. The expression vector according to embodiment 12, wherein the expression vector is derived from a lentiviral plasmid, e.g., pWPT-GFP-lenti-vector.

14. A virus comprising the vector according to embodiment 12 or 13.

15. A T cell to which the nucleic acid according to embodiment 11, the expression vector according to embodiment 12 or 13, or the virus according to embodiment 14 is transduced, wherein preferably, the T cell is a T lymphocyte.

16. A T cell expressing the chimeric antigen receptor according to any one of embodiments 1-10 on a surface thereof, wherein preferably, the T cell is a T lymphocyte.

17. A method for preventing or treating a tumor (e.g., cancer) or providing anti-tumor immunity in a subject, comprising administering to the subject an effective amount of a cell comprising the chimeric antigen receptor according to any one of embodiments 1-10, or of the T cell according to embodiment 15 or 16.

18. The method according to embodiment 17, wherein a patient with the tumor (e.g., cancer) has CLDN18.2 (e.g., at an elevated level, e.g., at a nucleic acid or protein level), and preferably, the tumor is gastric or pancreatic cancer.

19. The method according to embodiment 17 or 18, wherein the cell is administered in combination with one or more other therapies, such as a therapeutic modality and/or an additional therapeutic agent, and preferably the therapeutic modality comprises a surgical treatment and/or radiotherapy; preferably, the additional therapeutic agent is selected from a chemotherapeutic agent, a cytotoxic agent, a vaccine, an additional antibody, an anti-infective active agent, a small molecule drug, and an immunomodulatory agent.

20. Use of the chimeric antigen receptor according to any one of embodiments 1-10 in the preparation of a T cell or a drug comprising the T cell for preventing or treating a tumor (e.g., cancer) or providing anti-tumor immunity in a subject.

21. The use according to embodiment 20, wherein a patient with the tumor (e.g., cancer) has CLDN18.2 (e.g., at an elevated level, e.g., at a nucleic acid or protein level), and preferably, the tumor is gastric or pancreatic cancer.

22. A method for producing a T cell comprising transducing to the T cell an expression vector comprising a nucleic acid encoding the CAR molecule according to any one of embodiments 1-10 or the nucleic acid according to embodiment 11, or the expression vector according to embodiment 12 or 13.

## BRIEF DESCRIPTION OF THE DRAWINGS

FIG. 1 shows CAR viral plasmid (A) with an intracellular signaling region of CD28 and CAR viral plasmid (B) with an intracellular signaling region of 4-1BB;

FIG. 2 shows a picture taken by an immunofluorescence microscope (OLYMPUS, IX53) to detect GFP-positive 293T cells;

FIG. 3 shows the proportion of GFP-positive 293T cells after 72 h of lentiviral packaging detected by a flow cytometer;

FIG. 4 shows the proportion of GFP-positive T cells detected by a flow cytometer;

FIG. 5 shows the killing effect of CAR-T on NUGC-4 tumor cells;

FIG. 6 shows the cytokine secretion level in the NUGC-4 cell killing system;

FIG. 7 shows the killing level of CAR-T on DNA-G cells;

FIG. 8 shows the cytokine secretion level in the DNA-G cell killing system;

FIG. 9 shows the CAR-T proliferation activity in the NUGC-4 cell killing system;

FIG. 10 shows the CAR-T proliferation activity in the DNA-G cell killing system;

Figure 11 shows the proportion of CAR-positive T cells detected by flow cytometry;

FIG. 12 shows the killing efficiency of CAR-T for Claudin18.2-positive tumor cells;

FIG. 13 shows the killing efficiency of CAR-T for Claudin18.2-negative 293T cells;

FIG. 14 shows the CAR-T proliferation activity in 293T cells, DNA-G, and NUGC-4 cell killing systems;

FIG. 15 shows the results of degranulation assays after 6 hours of co-incubation of CAR-T cells with target and non-target cells;

FIG. 16 shows the tumor inhibition activity of CAR-T in an NUGC-4 tumor-bearing NOG mouse model;

FIG. 17 shows changes in body weight of the mice;

FIG. 18 shows the binding of anti-CLDN18.2 antibodies to the gastric cancer cell line NUGC-4, the gastric cancer cell line KATO III-hCLDN18.2, and the pancreatic cancer cell line DAN-G-hCLDN18.2;

FIG. 19 shows the anti-tumor effect of anti-CLDN18.2 antibodies in the mouse model of pancreatic cancer;

FIG. 20 shows the anti-tumor effect of anti-CLDN18.2 antibodies in the mouse model of gastric cancer.

## DETAILED DESCRIPTION

### I. Definitions

Before the present invention is described in detail below, it should be understood that the present invention is not limited to the particular methodology, protocols, and reagents described herein, as these may vary. It should also be understood that the terminology used herein is only intended to describe specific embodiments rather than to limit the scope of the present invention, which will be limited only by the appended claims. Unless otherwise defined, any technical and scientific term used herein has the same meaning as commonly understood by those of ordinary skill in the art to which the present invention belongs.

For the purpose of explaining this specification, the following definitions will be used, and wherever appropriate, terms used in the singular form may further include the plural form, and vice versa. It should be understood that the terms used herein are for the purpose of describing specific embodiments only, and are not intended to be limiting.

The term "about" used in combination with a numerical value is intended to encompass the numerical values in a range from a lower limit less than the specified numerical value by 5% to an upper limit greater than the specified numerical value by 5%.

As used herein, the term "and/or" refers to any one of the options or any two or more of the options.

As used herein, the term "comprise" or "include" is intended to mean that the described elements, integers or steps are included, but not to the exclusion of any other elements, integers or steps. The term "comprise" or "include" used herein, unless otherwise specified, also encompasses the situation where the entirety consists of the described elements, integers or steps. For example, when referring to an antibody variable region "comprising" a particular sequence, it is also intended to encompass an antibody variable region consisting of the particular sequence.

The term "CLAUDIN" or "CLDN" used herein is the most important skeleton protein that determines the structures of tight junctions between cells, and it is involved in adhesion junctions and plays an important role in the metastasis and invasion of tumor cells. Claudin proteins are widely present in mammalian epithelial and endothelial cells, primarily on the lateral surface of epithelial cells and on the plasma membrane of basal cells. Different Claudin proteins have their respective specific expression in different tissues, wherein the Claudin18 (CLDN18) gene is located at 3q22.3, has a molecular weight of 24 kDa, comprises 261 amino acid residues, and is a member of the Claudins superfamily, and its protein structure comprises 2 extracellular loops and 4 transmembrane domains. Two subtypes of human CLDN18 or Claudin18 protein are Claudin18.1 or CLDN18.1 (UniProt ID: P56856-1) and Claudin18.2 or CLDN18.2 (UniProt ID: P56856-2), respectively. In the primary structural sequences of the two proteins, only the amino acid residues at some positions from the N-terminus signal peptide to the extracellular loop 1 (Loop1) structure are different, especially at the extracellular loop 1, CLDN18.1 and CLDN18.2 differ by only 8 amino acids. The intergeneric sequence homology of the two subtypes of CLDN18 protein is also very high. The sequences of the extracellular loop 1 of CLDN18.2 are completely identical in different species such as humans, mice and rhesus monkeys, while the homology of human and mouse CLDN18.2 proteins reaches 84%, indicating that the sequence of CLDN18.2 protein is extremely conserved (O. Tureci. et al., Gene, 481:83-92, 2011). CLDN18.2 or any variants and isoforms thereof may be isolated from cells or tissues in which they are naturally expressed, or recombinantly produced using techniques well known in the art and/or those described herein. In one embodiment, the CLDN18.2 as described herein is human CLDN18.2.

The term "anti-CLDN18.2 antibody", "anti-CLDN18.2", "CLDN18.2 antibody" or "antibody that binds to CLDN18.2" used herein refers to an antibody capable of binding to (human) CLDN18.2 with sufficient affinity such that the antibody can be used as a therapeutic agent targeting (human) CLDN18.2. In one embodiment, the (human) CLDN18.2 antibody binds to (human) CLDN18.2 with high affinity *in vitro* or *in vivo*. In one embodiment, the (human) CLDN18.2 antibody does not bind to CLDN18.1. In one embodiment, the (human) CLDN18.2 antibody binds to cells expressing CLDN18.2 but does not bind to cells expressing CLDN18.1. In some embodiments, the binding is determined, e.g., by radioimmunoassay (RIA), bio-layer interferometry (BLI), MSD assay, surface plasmon resonance (SPR) or flow cytometry.

The term "chimeric antigen receptor" or alternatively "CAR" refers to a set of polypeptides, typically two sets in the simplest embodiment, which, when in an immune effector cell, provide the cell with specificity for a target cell (typically a cancer cell) and have intracellular signal production. In certain embodiments, CAR comprises at least an extracellular binding region, a transmembrane region, and an intracellular signaling region. In certain aspects, the sets of polypeptides are contiguous to each other.

The term "extracellular binding region" refers to a portion, of the chimeric antigen receptor, that recognizes the antigen, and in some embodiments, the extracellular binding region is an scFv, or dAb (domain antibody) or VHH from a monoclonal antibody.

The term "transmembrane region" refers to a portion, of the chimeric antigen receptor, that spans the membrane. The transmembrane region may be any protein structure that is thermodynamically stable in the membrane. This is typically an alpha helix comprising several hydrophobic residues. The transmembrane region of any transmembrane protein may be used to provide the transmembrane portion of the chimeric receptor. For example, the transmembrane region may be a transmembrane region from CD8α or CD28, and preferably comprises an amino acid sequence of SEQ ID NO: 80 or 86 or a sequence having at least 90%, 95%, 96%, 97%, 98% or 99% or more identity thereto.

The term "intracellular signaling region" refers to the functional portion of a protein that acts by transmitting information within a cell to regulate cellular activity via a defined signaling pathway by generating a second messenger or functioning as an effector by responding to such a messenger.

The term "CD3-ζ" is defined as a protein as provided by GenBan Acc.No. BAG36664.1 or an equivalent residue from a non-human species (e.g., mice, rodents, monkeys, apes, etc.), and "CD3-ζ signaling domain" is defined as an amino acid residue from the cytoplasmic domain of CD3-ζ or a functional derivative thereof, sufficient to functionally transmit the initiation signals required for T cell activation. In one aspect, a "CD3-ζ signaling domain" comprises a sequence as provided in SEQ ID NO: 82 or a sequence having at least 90%, 95%, 96%, 97%, 98%, or 99% or more identity thereto.

The term "4-1BB" refers to members of the TNFR superfamily having an amino acid sequence as provided in GenBank Acc.No.AAA62478.2, or equivalent residues from a non-human species such as mice, rodents, monkeys, and apes. In one aspect, "4-1BB co-stimulatory domain" is defined as amino acid residues 214-255 of GenBank Acc.No. AAA62478.2, or equivalent residues from a non-human species such as mice, rodents, monkeys, and apes. In one aspect, "4-1BB co-stimulatory domain" comprises a sequence as provided in SEQ ID NO: 87 or a sequence having at least 90%, 95%, 96%, 97%, 98%, or 99% or more identity thereto.

The term "CD28" refers to the human leukocyte differentiation antigen 28, which has an official name of CD28, and an ID number of 940 at NCBI GenBank, and has 3 isoforms (cDNA sequence/protein sequence), namely NM_006139.3/NP_006130.1, NM_001243077.1/NP_001230006.1, and NM_001243078.1/NP_001230007.1. As used herein, the term "CD28 co-stimulatory domain" comprises a sequence as provided in SEQ ID NO: 81 or a sequence having at least 90%, 95%, 96%, 97%, 98%, or 99% or more identity thereto.

The term "lentivirus" refers to a species of the retrovirus family. Lentiviruses are unique among retroviruses because they are capable of infecting non-dividing cells; they can deliver a large amount of genetic information into the DNA of a host cell, and thus this is one of the most efficient methods of gene delivery vectors. HIV, SIV and FIV are examples of lentiviruses. The term "lentiviral vector" refers to a vector derived from at least a portion of the lentiviral genome, in particular including a self-inactivating lentiviral vector. The lentiviral vector is available and known to those skilled in the art.

The term "antibody fragment" includes a portion of an intact antibody. In a preferred embodiment, the antibody fragment is an antigen binding fragment.

"Antigen binding fragment" refers to a molecule different from an intact antibody, which comprises a portion of the intact antibody and binds to an antigen to which the intact antibody binds. Examples of the antibody fragment include, but are not limited to, Fv, Fab, Fab', Fab'-SH, F(ab')$_2$, a domain antibody (dAb), a linear antibody, a single-chain antibody (e.g., scFv), a single-domain antibody (e.g., VHH), a bi-valent antibody or a fragment thereof, or a camelid antibody. The term "scFv" refers to a fusion protein comprising at least one antibody fragment comprising a variable region of a light chain and at least one antibody fragment comprising a variable region of a heavy chain, wherein the light and heavy chain variable regions are contiguously linked (e.g., via a synthetic linker, e.g., a short flexible polypeptide linker), and capable of being expressed as a single chain polypeptide, and the scFv retains the specificity of the intact antibody from which it is derived. Unless specified, the scFv, as used herein, may have the VL and VH variable regions in any order (e.g., relative to the N-terminus and C-terminus of the polypeptide), and may comprise a VL-linker-VH or a VH-linker-VL.

"Complementarity determining region" or "CDR region" or "CDR" is a region in an antibody variable domain that is highly variable in sequence and forms a structurally defined loop ("hypervariable loop") and/or comprises antigen-contacting residues ("antigen contact site"). CDRs are primarily responsible for binding to antigen epitopes. The CDRs of the heavy and light chains are generally referred to as CDR1, CDR2, and CDR3, and are numbered sequentially from the N-terminus. The CDRs located in the heavy chain variable domain of the antibody are referred to as HCDR1, HCDR2 and HCDR3, whereas the CDRs located in the light chain variable domain of the antibody are referred to as LCDR1, LCDR2 and LCDR3. In a given amino acid sequence of a light chain variable region or a heavy chain variable region, the exact amino acid sequence boundary of each CDR may be determined using any one or a combination of many well-known antibody CDR assignment systems including, e.g., Chothia based on the three-dimensional structure of antibodies and the topology of the CDR loops (Chothia et al. (1989) Nature, 342: 877-883; Al-Lazikani et al., Standard conformations for the canonical structures of immunoglobulins, Journal of Molecular Biology, 273: 927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

For example, according to different CDR determination schemes, the residues of each CDR are as follows.

| CDR | Kabat scheme | AbM scheme | Chothia scheme | Contact scheme |
|---|---|---|---|---|
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |
| HCDR1 | H31-H35B | H26-H35B | H26-H32 | H30-H35B |
| (Kabat numbering system) | | | | |
| HCDR1 | H31-H35 | H26-H35 | \| H26-H32 | H30-H35 |
| (Chothia numbering system) | | | | |
| HCDR2 | H50-H65 | H50-H58 | H53-H55 | H47-H58 |
| HCDR3 | H95-H102 | H95-H102 | H96-H101 | H93-H101 |
| (Kabat numbering system) | | | | |

CDRs can also be determined based on having the same Kabat numbering positions as a reference CDR sequence (e.g., any of the exemplary CDRs of the present invention).

Unless otherwise stated, the term "CDR" or "CDR sequence" used herein encompasses CDR sequences determined by any one of the schemes above.

Unless otherwise stated, residue positions of an antibody variable region (including heavy chain variable region residues and light chain variable region residues) used herein are numbered according to the Kabat numbering system (Kabat et al., Sequences of Proteins of Immunological Interest, 5th Ed., Public Health Service, National Institutes of Health, Bethesda, Md. (1991)).

In one embodiment, the heavy chain variable region CDRs of the antibody of the present invention are determined according to the following rules:

VH CDR1 is determined according to AbM rules, and VH CDR2 and VH CDR3 are both determined according to Kabat rules.

In one embodiment, the light chain variable region CDRs of the antibody of the present invention are determined according to Kabat rules.

In one embodiment, the heavy chain variable region CDRs of the antibody of the present invention are determined according to the following rules: VH CDR1 is determined according to AbM rules, and VH CDR2 and VH CDR3 are both determined according to Kabat rules; the light chain variable region CDRs are determined according to Kabat rules.

It should be noted that boundaries of CDRs in variable regions of the same antibody obtained based on different assignment systems may vary. That is, the CDR sequences of variable regions of the same antibody defined by different assignment systems differ. Accordingly, when it comes to defining an antibody with specific CDR sequences defined in the present invention, the scope of the antibody further encompasses such antibodies whose variable region sequences comprise the specific CDR sequences, but have claimed CDR boundaries different from the specific CDR boundaries defined in the present invention due to a different scheme (e.g., different assignment system rules or their combinations) applied.

Antibodies with different specificities (i.e., different binding sites for different antigens) have different CDRs (under the same assignment system). However, although CDRs vary from antibody to antibody, only a limited number of amino acid positions within the CDRs are directly involved in antigen binding. The smallest overlapping region may be determined using at least two of the Kabat, Chothia, AbM, Contact, and North methods, thereby providing a "minimal binding unit" for antigen binding. The minimal binding unit may be a sub-portion of the CDRs. As will be clear to those skilled in the art, residues in remaining portions of the CDR sequences may be determined by the structure and protein folding of the antibody. Accordingly, variants of any CDR provided herein are also considered in the present invention. For example, in a variant of one CDR, the amino acid residue of the minimal binding unit may remain unchanged, while the remaining CDR residues defined according to Kabat or Chothia may be substituted with conservative amino acid residues.

"Humanized" antibody refers to an antibody comprising amino acid residues from non-human CDRs and amino acid residues from human FRs. In some embodiments, a humanized antibody will comprise at least one, or generally two of substantially all variable domains, wherein all or substantially all CDRs (e.g., CDRs) correspond to those of a non-human antibody, and all or substantially all FRs correspond to those of a human antibody. A humanized antibody may optionally comprise at least a portion of an antibody constant region derived from a human antibody. The "humanized form" of an antibody (e.g., a non-human antibody) refers to an antibody that has been humanized.

"Human antibody", "fully human antibody" and "fully humanized antibody" are used interchangeably, and refer to an antibody having an amino acid sequence which corresponds to the amino acid sequence of an antibody generated by a human or human cell or derived from a non-human source that utilizes human antibody libraries or other human antibody encoding sequences. This definition of a human antibody explicitly excludes humanized antibodies comprising non-human antigen binding residues.

As used herein, the term "binding" or "specific binding" means that the binding effect is selective for antigens and may be distinguished from unwanted or non-specific interactions. The ability of an antigen-binding site to bind to a particular antigen may be determined by enzyme-linked immunosorbent assay (ELISA) or conventional binding assays known in the art, such as radioimmunoassay (RIA), bio-layer interferometry, MSD assay or surface plasmon resonance (SPR).

The term "therapeutic agent" described herein encompasses any substance that is effective in preventing or treating a tumor, e.g., cancer, including a chemotherapeutic agent, a cytokine, a cytotoxic agent, an additional antibody, a small molecule drug or an immunomodulatory agent (e.g., an immunosuppressant).

The term "cytotoxic agent" used herein refers to a substance that inhibits or prevents cell functions and/or causes cell death or cell destruction.

"Chemotherapeutic agents" include chemical compounds useful in the treatment of cancer.

The term "small molecule drug" refers to a low molecular weight organic compound capable of regulating biological processes. "Small molecule" is defined as a molecule with a molecular weight of less than 10 kD, usually less than 2 kD and preferably less than 1 kD. The small molecule includes but is not limited to inorganic molecules, organic molecules, organic molecules containing inorganic components, molecules containing radioactive atoms, synthetic molecules, peptide mimetics, and antibody mimetics. As therapeutic agents, small molecules penetrate cells better, are less susceptible to degradation and are less likely to induce an immune response compared with large molecules.

The term "immunomodulatory agent" used herein refers to a natural or synthetic active agent or drug that suppresses or modulates an immune response. The immune response may be a humoral response or a cellular response. The immunomodulatory agent includes an immunosuppressant.

"Immunosuppressant", "immunosuppressive drug" or "immunosuppressive substance" used herein refers to a therapeutic agent used in immunosuppressive therapy to suppress or prevent the activity of the immune system.

The term "effective amount" refers to the amount or dose of the antibody, fragment, conjugate, composition or combination of the present invention that generates expected effects in a patient in need of treatment or prevention after administration to the patient in a single or multiple doses.

"Therapeutically effective amount" refers to an amount effective to achieve a desired therapeutic result at a necessary dose for a necessary period of time. The therapeutically effective amount is also such an amount that any toxic or adverse effect of the antibody or the fragment thereof, or conjugate, composition or combination thereof is inferior to the therapeutically beneficial effect. The "therapeutically effective amount" preferably inhibits a measurable parameter (e.g., tumor volume) by at least about 20%, more preferably by at least about 40%, and even more preferably by at least about 50%, 60% or 70%, relative to untreated subjects.

"Prophylactically effective amount" refers to an amount effective to achieve a desired prophylactic result at a necessary dose for a necessary period of time. Generally, since a prophylactic dose is administered in a subject before or at an earlier stage of a disease, the prophylactically effective amount will be less than the therapeutically effective amount.

"Individual" or "subject" includes mammals. The mammals include, but are not limited to, domestic animals (e.g., cattle, goats, cats, dogs, and horses), primates (e.g., human and non-human primates such as monkeys), rabbits, and rodents (e.g., mice and rats). In some embodiments, the individual or subject is a human.

The calculation of sequence identity between sequences is performed as follows.

To determine the percent identity of two amino acid sequences or two nucleic acid sequences, the sequences are aligned for optimal comparison purposes (e.g., for optimal alignment, gaps may be introduced in one or both of the first and second amino acid sequences or nucleic acid sequences, or non-homologous sequences may be discarded for comparison). In one preferred embodiment, for comparison purposes, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm may be used to compare two sequences and calculate the percent identity between the sequences. In one preferred embodiment, the percent identity between two amino acid sequences is determined with the Needlema and Wunsch algorithm ((1970) J. Mol. Biol., 48:444-453; available at http://www.gcg.com) which has been integrated into the GAP program of the GCG software package, using the Blossom 62 matrix or PAM250 matrix and gap weight of 16, 14, 12, 10, 8, 6, or 4 and length weight of 1, 2, 3, 4, 5, or 6. In another preferred embodiment, the percent identity between two nucleotide acid sequences is determined with the GAP program of the GCG software package (available at http://www.gcg.com), using the NWSgapdna.CMP matrix and a gap weight of 40, 50, 60, 70, or 80 and a length weight of 1, 2, 3, 4, 5, or 6. A particularly preferred parameter set (and one that should be used unless otherwise stated) is a Blossom 62 scoring matrix with a gap penalty of 12, a gap extension penalty of 4, and a frameshift gap penalty of 5. The percent identity between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4:11-17) which has been incorporated into the ALIGN program (version 2.0). Additionally or alternatively, the nucleic acid sequences and protein sequences described herein may be further used as "query sequences" to perform searches against public databases to, e.g., identify other family member sequences or related sequences.

The term "anti-tumor effect" refers to a biological effect that may be demonstrated by a variety of means, including but not limited to, for example, decrease in tumor volume, decrease in number of tumor cells, decrease in tumor cell proliferation, or decrease in tumor cell viability.

The terms "tumor" and "cancer" are used interchangeably herein and encompass solid and liquid tumors.

The terms "cancer" and "cancerous" refer to or describe a physiological disease in mammals that is typically characterized by unregulated cell growth. In certain embodiments, cancers suitable for treatment with the antibody of the present invention include gastric cancer or pancreatic cancer, including metastatic forms of such cancers.

The term "tumor" refers to all neoplastic cell growth and proliferation, whether being malignant or benign, and all pre-cancerous and cancerous cells and tissues. The terms "cancer", "cancerous" and "tumor" are not mutually exclusive when referred to herein.

The term "combination therapy" refers to the administration of two or more therapeutic agents and/or modalities (e.g., radiotherapy or surgery) to treat the diseases described herein. Such administration includes co-administration of these therapeutic agents in a substantially simultaneous manner, for example, in a single capsule with a fixed proportion of active ingredients. Alternatively, such administration includes co-administration of the active ingredients in a variety of or separate containers (e.g., tablets, capsules, powder and liquid). The powder and/or liquid may be reconstituted or diluted to a desired dose before administration. In addition, such administration further includes using each type of the therapeutic agents at approximately the same time or in a sequential manner at different times. In any case, the therapeutic regimen will provide the beneficial effect of the pharmaceutical combination in the treatment of disorders or symptoms described herein.

As used herein, "treatment" (or "treat" or "treating") refers to slowing, interrupting, arresting, alleviating, stopping, lowering, or reversing the progression or severity of an existing symptom, disorder, condition, or disease.

As used herein, "prevention" (or "prevent" or "preventing") includes the inhibition of the occurrence or development of symptoms of a disease or disorder, or a specific disease or disorder. In some embodiments, subjects with a family history of cancer are candidates for preventive regimens. Generally, in the context of cancer, the term "prevention" refers to the administration of a drug prior to the onset of signs or symptoms of cancer, particularly in subjects at risk of cancer.

The term "vector" used herein refers to a nucleic acid molecule capable of proliferating another nucleic acid to which it is linked. The term includes vectors that serve as self-replicating nucleic acid structures as well as vectors binding to the genome of a host cell into which they have been introduced. Some vectors are capable of directing the expression of a nucleic acid to which they are operably linked. Such vectors are called "expression vectors" herein.

**II. Chimeric antigen receptor and nucleic acid encoding same**

In one aspect of the present invention, the present invention relates to an isolated chimeric antigen receptor (CAR) molecule comprising (e.g., sequentially linked) an extracellular binding region binding to CLDN18.2, a transmembrane region, and an intracellular signaling region (e.g., comprising a co-stimulatory domain).

**II-1 Extracellular binding domain**

In one embodiment, the extracellular binding region binding to CLDN18.2 described herein binds to wild-type CLDN18.2, e.g., human wild-type CLDN18.2.

The extracellular binding region binding to CLDN18.2 may be any antigen binding domain binding to CLDN18.2: including but not limited to monoclonal, polyclonal, recombinant, human, and humanized antibodies, and functional fragments thereof, including but not limited to single-domain antibodies such as heavy chain variable domains (VH), light chain variable domains (VL) and variable domains of camelid-derived nanobodies (VHH), and function as antigen binding domains in conjunction with alternative scaffolds known in the art. In certain cases, it is advantageous for the antigen binding domain to be derived from the species with the same use as the ultimate use of the CAR. For example, for use in humans, it may be advantageous for the antigen binding domain of the CAR to comprise residues of the human or humanized antigen binding domain for an antibody or antibody fragment. Thus, in one aspect, the antigen binding domain comprises a human antibody or an antibody fragment. In one aspect, the antigen binding domain is an scFv.

In one embodiment, the extracellular binding region binding to CLDN18.2 comprises one or more (e.g., all 3) of the light chain complementarity determining region 1 (LCDR1), light chain complementarity determining region 2 (LCDR2), and light chain complementarity determining region 3 (LCDR3) described herein and one or more (e.g., all 3) of the heavy chain complementarity determining region 1 (HCDR1), heavy chain complementarity determining region 2 (HCDR2), and heavy chain complementarity determining region 3 (HC CDR3) described herein.

In some embodiments, the extracellular binding region binding to CLDN18.2 of the present invention comprises 3 complementarity determining regions (HCDRs) from a heavy chain variable region: HCDR1, HCDR2 and HCDR3.

In some embodiments, the extracellular binding region binding to CLDN18.2 of the present invention comprises 3 complementarity determining regions (LCDRs) from the light chain variable region: LCDR1, LCDR2 and LCDR3.

In some embodiments, the extracellular binding region binding to CLDN18.2 of the present invention comprises 3 complementarity determining regions (HCDRs) from the heavy chain variable region and 3 complementarity determining regions (LCDRs) from the light chain variable region.

In some aspects, the extracellular binding region binding to CLDN18.2 of the present invention comprises a heavy chain variable region (VH). In some aspects, the extracellular binding region binding to CLDN18.2 of the present invention comprises a light chain variable region (VL). In some aspects, the extracellular binding region binding to CLDN18.2 of the present invention comprises a heavy chain variable region and a light chain variable region. In some embodiments, the heavy chain variable region comprises 3 complementarity determining regions (CDRs) from the heavy chain variable region: HCDR1, HCDR2 and HCDR3. In some embodiments, the light chain variable region comprises 3 complementarity determining regions (CDRs) from the light chain variable region: LCDR1, LCDR2 and LCDR3.

In some embodiments, the heavy chain variable region of the present invention

(i) comprises or consists of an amino acid sequence having at least 90%, 91%, 92%, 93%, 94%, 95%, 96%, 97%, 98%, or 99% identity to an amino acid sequence selected from SEQ ID NO: 4, 16, 24, 86, 32, 41, 49, 57, 64, and 71; or
(ii) comprises or consists of an amino acid sequence selected from SEQ ID NO: 4, 16, 24, 86, 32, 41, 49, 57, 64, and 71; or
(iii) comprises or consists of an amino acid sequence having 1 or more (preferably not more than 10, and more preferably not more than 5, 4, 3, 2, or 1) amino acid changes (preferably amino acid replacements, and more preferably amino acid conservative replacements) as compared to an amino acid sequence selected from SEQ ID NO: 4, 16, 24, 86, 32, 41, 49, 57, 64, and 71, wherein preferably, the amino acid alterations do not occur in the CDRs.

In some embodiments, the VH region comprises a back mutation in the FR region, for example, to increase antibody production, e.g., a substitution of F to V at position H78 (Kabat numbering position).

In some embodiments, the light chain variable region of the present invention

(i) comprises or consists of an amino acid sequence having at least 90%, 91%, 92%, 93%, 94%, 95%, 96%, 97%, 98%, or 99% identity to an amino acid sequence selected from SEQ ID NO: 9, 19, 27, 36, 87, 44, 52, 59, 66, and 75; or

(ii) comprises or consists of an amino acid sequence selected from SEQ ID NO: 9, 19, 27, 36, 87, 44, 52, 59, 66, and 75; or

(iii) comprises or consists of an amino acid sequence having 1 or more (preferably not more than 10, and more preferably not more than 5, 4, 3, 2, or 1) amino acid changes (preferably amino acid replacements, and more preferably amino acid conservative replacements) as compared to an amino acid sequence selected from SEQ ID NO: 9, 19, 27, 36, 87, 44, 52, 59, 66, and 75, wherein preferably, the amino acid alterations do not occur in the CDRs.

In some embodiments, the 3 complementarity determining regions (HCDRs) from the heavy chain variable region of the present invention, HCDR1, HCDR2 and HCDR3, are selected from

(i) three complementarity determining regions HCDR1, HCDR2 and HCDR3 contained in the VH set forth in SEQ ID NO: 4, 16, 24, 86, 32, 41, 49, 57, 64, or 71, and

(ii) a sequence comprising a total of at least one and no more than 5, 4, 3, 2, or 1 amino acid change (preferably amino acid replacement, and more preferably conservative replacement) in the three HCDRs relative to any sequence of (i).

In some embodiments, the 3 complementarity determining regions (LCDRs) from the light chain variable region of the present invention, LCDR1, LCDR2, and LCDR3, are selected from

(i) three complementarity determining regions LCDR1, LCDR2 and LCDR3 contained in the VL set forth in SEQ ID NO: 9, 19, 27, 36, 87, 44, 52, 59, 66, or 75, and

(ii) a sequence comprising a total of at least one and not more than 5, 4, 3, 2, or 1 amino acid change (preferably amino acid replacement, and more preferably conservative replacement) in the three LCDRs relative to any sequence of (i).

In some embodiments, the HCDR1 comprises or consists of an amino acid sequence of SEQ ID NO: 1, 13, 21, 29, 38, 46, 54, 61, or 68, or the HCDR1 comprises an amino acid sequence having one, two or three changes (preferably amino acid replacements, and more preferably conservative replacements) as compared to the amino acid sequence of SEQ ID NO: 1, 13, 21, 29, 38, 46, 54, 61, or 68.

In some embodiments, the HCDR2 comprises or consists of an amino acid sequence of SEQ ID NO: 2, 14, 22, 88, 30, 39, 47, 55, 62, or 69, or the HCDR2 comprises an amino acid sequence having one, two or three changes (preferably amino acid replacements, and more preferably conservative replacements) as compared to the amino acid sequence of SEQ ID NO: 2, 14, 22, 88, 30, 39, 47, 55, 62, or 69.

In some embodiments, the HCDR3 comprises or consists of an amino acid sequence of SEQ ID NO: 3, 15, 23, 31, 40, 48, 56, 63, or 70, or the HCDR3 comprises an amino acid sequence having one, two or three changes (preferably amino acid replacements, and more preferably conservative replacements) as compared to the amino acid sequence of SEQ ID NO: 3, 15, 23, 31, 40, 48, 56, 63, or 70.

In some embodiments, the LCDR1 comprises or consists of an amino acid sequence of SEQ ID NO: 6, 33, 89, 42, 50, or 72, or the LCDR1 comprises an amino acid sequence having one, two or three changes (preferably amino acid replacements, and more preferably conservative replacements) as compared to the amino acid sequence of SEQ ID NO: 6, 33, 89, 42, 50, or 72.

In some embodiments, the LCDR2 comprises or consists of an amino acid sequence of SEQ ID NO: 7, 17, 25, 34, or 73, or the LCDR2 comprises an amino acid sequence having one, two or three changes (preferably amino acid replacements, and preferably conservative replacements) as compared to the amino acid sequence of SEQ ID NO: 7, 17, 25, 34, or 73.

In some embodiments, the LCDR3 comprises or consists of an amino acid sequence of SEQ ID NO: 8, 18, 26, 35, 43, 51, 58, 65, or 74, or the LCDR3 comprises an amino acid sequence having one, two or three changes (preferably amino acid replacements, and more preferably conservative replacements) as compared to the amino acid sequence of SEQ ID NO: 8, 18, 26, 35, 43, 51, 58, 65, or 74.

In certain embodiments, the replacements occur in a CDR region of the extracellular binding region of CLDN18.2. Generally, the obtained variant has modifications (e.g., improvements) in certain biological properties (e.g., increased affinity) relative to the parent antibody and/or will have certain substantially retained biological properties of the parent antibody. Exemplary replacement variants are affinity-matured antibodies. In some embodiments, the alteration of the HCDRs or LCDRs is a hot spot mutation to alter the binding affinity of the binding region. In some embodiments, the alteration is an alteration at the HCDR2 or LCDR1. In some embodiments, the alteration is at position H55 of the HCDR2 (Kabat numbering position), e.g., a substitution of G to V, and/or at position L27D of the LCDR1 (Kabat numbering position), e.g., a substitution of N to Q.

In some specific embodiments of the present invention, the extracellular binding region binding to CLDN18.2 of the present invention comprises a combination of HCDR1, HCDR2 and HCDR3 in the following table:

|       | SEQ ID NO |    |    |    |    |    |    |    |    |    |
|-------|-----------|----|----|----|----|----|----|----|----|----|
| HCDR1 | 1         | 13 | 21 | 21 | 29 | 38 | 46 | 54 | 61 | 68 |
| HCDR2 | 2         | 14 | 22 | 88 | 30 | 39 | 47 | 55 | 62 | 69 |
| HCDR3 | 3         | 15 | 23 | 23 | 31 | 40 | 48 | 56 | 63 | 70 |

In some specific embodiments of the present invention, the extracellular binding region binding to CLDN18.2 of the present invention comprises a combination of LCDR1, LCDR2 and LCDR3 in the following table:

|       | SEQ ID NO |    |    |    |    |    |    |    |    |    |
|-------|-----------|----|----|----|----|----|----|----|----|----|
| LCDR1 | 6         | 6  | 6  | 33 | 89 | 42 | 50 | 50 | 50 | 72 |
| LCDR2 | 7         | 17 | 25 | 34 | 34 | 17 | 17 | 17 | 17 | 73 |
| LCDR3 | 8         | 18 | 26 | 35 | 35 | 43 | 51 | 58 | 65 | 74 |

In some specific embodiments of the present invention, the extracellular binding region binding to CLDN18.2 of the present invention comprises a combination of HCDR1, HCDR2, HCDR3 and LCDR1, LCDR2 and LCDR3 in the following table:

|       | SEQ ID NO |    |    |    |    |    |    |    |    |    |    |
|-------|-----------|----|----|----|----|----|----|----|----|----|----|
| HCDR1 | 1         | 13 | 21 | 21 | 29 | 29 | 38 | 46 | 54 | 61 | 68 |
| HCDR2 | 2         | 14 | 22 | 88 | 30 | 30 | 39 | 47 | 55 | 62 | 69 |
| HCDR3 | 3         | 15 | 23 | 23 | 31 | 31 | 40 | 48 | 56 | 63 | 70 |
| LCDR1 | 6         | 6  | 6  | 6  | 33 | 89 | 42 | 50 | 50 | 50 | 72 |
| LCDR2 | 7         | 17 | 25 | 25 | 34 | 34 | 17 | 17 | 17 | 17 | 73 |
| LCDR3 | 8         | 18 | 26 | 26 | 35 | 35 | 43 | 51 | 58 | 65 | 74 |

In some specific particular embodiments of the present invention, the anti-CLDN18.2 antibody or the antigen binding fragment thereof of the present invention comprises a VH and a VL comprising or consisting of amino acid sequences shown below, respectively:

SEQ ID NOs: 4 and 9;
SEQ ID NOs: 16 and 19;
SEQ ID NOs: 24 and 27;
SEQ ID NOs: 86 and 27;
SEQ ID NOs: 32 and 36;
SEQ ID NOs: 32 and 87;
SEQ ID NOs: 41 and 44;
SEQ ID NOs: 49 and 52;
SEQ ID NOs: 57 and 59;
SEQ ID NOs: 64 and 66; or
SEQ ID NOs: 71 and 75.

In one embodiment of the present invention, the amino acid changes described herein include amino acid replacements, insertions or deletions. Preferably, the amino acid changes described herein are amino acid replacements, preferably conservative replacements.

In a preferred embodiment, the amino acid changes described herein occur in a region outside the CDRs (e.g., in FRs). More preferably, the amino acid changes described herein occur in a region outside the heavy chain variable region and/or outside the light chain variable region. In some embodiments, the amino acid changes described herein occur in the Fc region of the heavy chain constant region of the antibody.

In some embodiments, the replacement is a conservative replacement. A conservative replacement refers to the replacement of an amino acid by another amino acid of the same class, e.g., the replacement of an acidic amino acid by another acidic amino acid, the replacement of a basic amino acid by another basic amino acid, or the replacement of a neutral amino acid by another neutral amino acid. Exemplary replacements are shown in the table below:

| Original residues | Exemplary replacement | Preferred conservative amino acid replacement |
|---|---|---|
| Ala (A) | Val, Leu, Ile | Val |
| Arg (R) | Lys, Gln, Asn | Lys |
| Asn (N) | Gln, His, Asp, Lys, Arg | Gln |
| Asp (D) | Glu, Asn | Glu |
| Cys (C) | Ser, Ala | Ser |
| Gln (Q) | Asn, Glu | Asn |
| Glu (E) | Asp, Gln | Asp |
| Gly (G) | Ala | Ala |
| His (H) | Asn, Gln, Lys, Arg | Arg |
| Ile (I) | Leu, Ual, Met, Ala, Phe, Nle | Leu |
| Leu (L) | Nle, Ile, Ual, Met, Ala, Phe | Ile |
| Lys (K) | Arg, Gln, Asn | Arg |
| Met (M) | Leu, Phe, Ile | Leu |
| Phe (F) | Trp, Leu, Ual, Ile, Ala, Tyr | Tyr |
| Pro (P) | Ala | Ala |
| Ser (S) | Thr | Thr |
| Thr (T) | Val, Ser | Ser |
| Trp (W) | Tyr, Phe | Tyr |
| Tyr (Y) | Trp, Phe, Thr, Ser | Phe |
| Val (V) | Ile, Leu, Met, Phe, Ala, Nle | Leu |

In some embodiments, the extracellular binding region of CLDN18.2 of the present invention is an scFv fragment of the anti-CLDN18.2 antibody.

In some embodiments, the scFv fragment comprises a linker. In some embodiments, the linker is a peptide linker. The peptide linker may or may not predominantly comprise the following amino acid residues: Gly, Ser, Ala or Thr. Useful linkers include glycine-serine polymers including, for example, (GS)n, (GSGGS)n, (GGGGS)n, (GGGS)n and (GGGGS)nG, wherein n is an integer of at least 1 (and preferably 2, 3, 4, 5, 6, 7, 8, 9, or 10). In some embodiments, the linker comprises an amino acid sequence of SEQ ID NO: 12.

In some embodiments, the scFv fragment comprises or consists of an amino acid sequence selected from SEQ ID NOs: 11, 20, 28, 37, 45, 53, 60, 67, and 76, or an amino acid sequence having at least 90%, 91%, 92%, 93%, 94%, 95%, 96%, 97%, 98%, or 99% identity to the amino acid sequence.

## II-2 Transmembrane region

In various embodiments, with respect to the transmembrane region, the CAR may be designed to include a transmembrane region linked to the extracellular domain of the CAR. The transmembrane region may comprise one or more additional amino acids contiguous with the transmembrane region, for example, one or more amino acids (e.g., 1, 2, 3, 4, 5, 6, 7, 8, 9, 10 up to 15 amino acids of the extracellular region) associated with the extracellular region of a protein from which the transmembrane is derived and/or one or more additional amino acids (e.g., 1, 2, 3, 4, 5, 6, 7, 8, 9, 10 up to 15 amino acids of the intracellular region) associated with the extracellular region of the protein from which the transmembrane protein is derived. In one aspect, the transmembrane region is a region associated with one of the other regions of the CAR used, e.g., in one embodiment, the transmembrane region may be from the same protein from which the signaling domain, co-stimulatory domain, or hinge region is derived. In another aspect, the transmembrane region is not the same protein derived from any other region of the CAR. In certain instances, the transmembrane region may be selected or modified by amino acid substitutions to avoid binding of such regions to transmembrane regions of the same or different surface membrane proteins, e.g., to minimize the interaction with other members of the receptor complex. In one aspect, the transmembrane region is capable of homodimerizing with another CAR on the cell surface of the CAR-expressing cell. In a different aspect, the amino acid sequence of the transmembrane region may be modified or substituted so as to minimize the interaction with the binding domain of a native binding partner present in a cell expressing the same CAR.

The transmembrane region may be derived from natural or recombinant sources.

When the source is natural, the domain may be derived from any membrane-bound or transmembrane protein. In one aspect, the transmembrane region is capable of signaling to the intracellular region whenever the CAR binds to a target. The transmembrane region particularly used in the present invention may include at least the following transmembrane regions: e.g., CD28, CD8 (e.g., CD8$\alpha$, CD8$\beta$). In one embodiment, the transmembrane domain comprises a transmembrane domain described herein, e.g., comprising or consisting of a sequence of SEQ ID NO: 80 or 86, an amino acid sequence having at least 1, 2 or 3 modifications (e.g., substitutions) but not more than 20, 10 or 5 modifications (e.g., substitutions, such as conservative substitutions) relative to the amino acid sequence of SEQ ID NO: 80 or 86, or a sequence having at least 90%, 91% or 95% identity to the amino acid sequence of SEQ ID NO: 80 or 86.

In certain instances, the transmembrane region may be linked to the extracellular region of the CAR, e.g., the antigen binding domain of the CAR, via a hinge (e.g., a hinge from a human protein). For example, in one embodiment, the hinge may be a human Ig (immunoglobulin) hinge (e.g., an IgG4 hinge, an IgD hinge), a GS linker (e.g., the GS linker described herein), a KIR2DS2 hinge, or a CD8 hinge. In one embodiment, the hinge region comprises (e.g., consists of) an amino acid sequence of SEQ ID NO: 79, or a sequence having at least 90%, 91%, 92%, 93%, 94%, 95%, 96%, or 97% identity to the amino acid sequence of SEQ ID NO: 79.

**II-3 Intracellular signaling region**

In some embodiments, the CAR molecule comprises an intracellular signaling region. In some embodiments, the intracellular signaling region comprises a co-stimulatory domain. In one embodiment, the co-stimulatory domain is a functional signaling domain derived from a protein selected from the group consisting of CD28 and 4-1BB (CD137) and a functional variant thereof. In one embodiment, the co-stimulatory domain comprises or consists of a sequence of SEQ ID NO: 81 or 87. In one embodiment, the co-stimulatory domain comprises or consists of an amino acid sequence having at least 1, 2 or 3 modifications (e.g., substitutions) but not more than 20, 10 or 5 modifications (e.g., substitutions, such as conservative substitutions) relative to an amino acid sequence of SEQ ID NO: 81 or 87, or a sequence having 90%, 91%, 92%, 93%, 94%, 95%, 96%, or 97% identity to the amino acid sequence of SEQ ID NO: 81 or 87.

The intracellular signaling region is generally responsible for the activation of at least one of the normal effector functions of the immune cell into which the CAR has been introduced. The term "effector function" refers to a specialized function of a cell. The effector function of a T cell may be, for example, cytolytic activity or helper activity, including secretion of cytokines. Thus, the term "intracellular signaling region" refers to a portion of a protein that transduces an effector function signal and directs a cell to perform a particular function. Although it is generally possible to use the entire intracellular signaling region, it is unnecessary to use the entire strand in many cases. In terms of using truncated portions of the intracellular signaling region, such truncated portions can be used to replace the complete chain, provided that they transduce effector function signals. Thus, the term "intracellular signaling region" is meant to include a truncated portion of the intracellular signaling region sufficient to transduce an effector function signal.

The intracellular signaling region of the CAR used in the present invention further includes the cytoplasmic sequences of the T cell receptor (TCR) and co-receptor that act together to initiate signal transduction following antigen receptor engagement, as well as any derivative or variant of these sequences and any recombinant sequence with the same functional capability. It is well known that the signal generated by the TCR alone is insufficient to fully activate the T cell and secondary and/or co-stimulatory signals are also required. Thus, T cell activation may be referred to as mediated by two different kinds of cytoplasmic signaling sequences: those that elicit antigen-dependent primary activation through the TCR (primary intracellular signaling regions, e.g., signaling domains), and those that function in an antigen-independent manner to provide a secondary or co-stimulatory signal (secondary cytoplasmic domains, e.g., co-stimulatory domains). The primary cytoplasmic signaling domain regulates primary activation of the TCR complex either in a stimulatory manner or in an inhibitory manner. The primary intracellular signaling region that functions in a stimulatory manner may contain signaling motifs referred to as immunoreceptor tyrosine-based activation motifs or ITAMs. Examples of ITAM-containing signaling domains particularly used in the present invention include CD3$\zeta$. In one embodiment, the intracellular signaling region of the CAR of the present invention comprises, e.g., a CD3-$\zeta$ signaling domain. In one aspect, the CD3-$\zeta$ signaling domain comprises or consists of a sequence set forth in SEQ ID NO: 82 or an amino acid sequence having at least 90%, 91%, 92%, 93%, 94%, 95%, 96%, 97%, 98% or 99% identity thereto.

Thus, the intracellular signaling region of the CAR of the present invention may comprise a CD3-ζ signaling domain, or a combination of a CD3-ζ signaling domain and other intracellular signaling regions, e.g., a co-stimulatory domain, for the CAR of the present invention. For example, the intracellular signaling region of the CAR may include a CD3ζ chain portion and a CD28 or 4-1BB co-stimulatory domain, and preferably, the co-stimulatory domain is located before the CD3ζ signaling domain.

The intracellular signaling regions within the cytoplasmic portion of the CAR of the present invention may be linked to each other in random or in a specified order. Optionally, a short oligopeptide or polypeptide linker, e.g., in a length between 2 and 10 amino acids(e.g., 2, 3, 4, 5, 6, 7, 8, 9, or 10 amino acids) can form a linkage between intracellular signaling regions. In one embodiment, a glycine--serine doublet may be used as a suitable linker. In one embodiment, a single amino acid such as alanine or glycine may be used as a suitable linker.

In one aspect, the intracellular signaling region is designed to comprise two or more, e.g., 2, 3, 4, 5 or more co-stimulatory signaling domains. In one embodiment, two or more, e.g., 2, 3, 4, 5 or more, co-stimulatory signaling domains are separated by a linker (e.g., the linker described herein) molecule. In one embodiment, the intracellular signaling region comprises two co-stimulatory signaling domains. In certain embodiments, the linker molecule is a glycine residue.In certain embodiments, the linker is an alanine residue.

In one aspect, the intracellular signaling region is designed to comprise a signaling domain of CD3-ζ and a CD28 co-stimulatory domain. In one aspect, the intracellular signaling region is designed to comprise a signaling domain of CD3-ζ and a 4-1BB co-stimulatory domain. In one aspect, the 4-1BB co-stimulatory domain comprises or consists of a sequence set forth in SEQ ID NO: 87 or an amino acid sequence having at least 90%, 91%, 92%, 93%, 94%, 95%, 96% or 97% identity thereto. In one aspect, the CD28 co-stimulatory domain comprises or consists of a sequence set forth in SEQ ID NO: 81 or an amino acid sequence having at least 90%, 91%, 92%, 93%, 94%, 95%, 96% or 97% identity thereto. In one aspect, the signaling domain of CD3-ζ comprises or consists of a sequence set forth in SEQ ID NO: 82 or an amino acid sequence having at least 90%, 91%, 92%, 93%, 94%, 95%, 96%, 97%, 98% or 99% identity thereto.

The CAR molecule may further comprise a signal peptide, e.g., a CD8 signal peptide, e.g., comprising or consisting of an amino acid sequence of SEQ ID NO: 78, and preferably, the signal peptide is located at the N-terminus of the extracellular binding region binding to CLDN18.2.

The CAR molecule may further comprise at the C-terminus a reporter gene, e.g., EGFP, e.g., comprising a sequence set forth in SEQ ID NO: 84 or an amino acid sequence having at least 90%, 91%, 92%, 93%, 94%, 95%, 96%, 97%, 98%, or 99% identity thereto, and preferably, the CAR molecule further comprises a self-cleaving peptide linked to the reporter gene, e.g., T2A, facilitating cleavage of the reporter gene from the CAR molecule, and e.g., comprising or consisting of an amino acid sequence of SEQ ID NO: 83.

Accordingly, the CAR molecule of the present invention comprises a signal peptide, e.g., comprising or consisting of an amino acid sequence of SEQ ID NO: 78; an extracellular binding region binding to CLDN18.2, e.g., an scFv binding to CLDN18.2, e.g., comprising or consisting of an amino acid sequence selected from SEQ ID NOs: 11, 20, 28, 37, 45, 53, 60, 67, and 76, or an amino acid sequence having at least 90%, 91%, 92%, 93%, 94%, 95%, 96%, 97%, 98%, or 99% identity to the amino acid sequence; a hinge region, e.g., a CD8 hinge region, e.g., comprising or consisting of an amino acid sequence of SEQ ID NO: 79; a transmembrane region, e.g., a CD28 transmembrane region or CD8α transmembrane region, e.g., comprising or consisting of an amino acid sequence of SEQ ID NO: 80 or 86; an intracellular signaling region comprising or consisting of a CD28 co-stimulatory domain (e.g., comprising or consisting of a sequence set forth in SEQ ID NO: 81 or an amino acid sequence having at least 90%, 91%, 92%, 93%, 94%, 95%, 96%, or 97% identity thereto) or a 4-BB co-stimulatory domain (e.g., comprising or consisting of a sequence set forth in SEQ ID NO: 87 or an amino acid sequence having at least 90%, 91%, 92%, 93%, 94%, 95%, 96%, or 97% identity thereto), and CD3ζ (e.g., comprising a sequence set forth in SEQ ID NO: 82 or an amino acid sequence having at least 90%, 91%, 92%, 93%, 94%, 95%, 96%, 97%, 98%, or 99% identity thereto); and optionally, a self-cleaving peptide, e.g., T2A, facilitating cleavage of a reporter gene, and a reporter gene, e.g., EGFP.

## III. Encoding nucleic acid and vector

The present invention provides a nucleic acid encoding a CAR comprising a nucleic acid sequence encoding one or more CARs of the present invention. In one aspect, a CAR-encoding nucleic acid is provided in the form of a DNA construct.

The CAR-encoding nucleic acid may comprise a Kozak sequence at the 5' end to promote stability and translation efficiency. In one embodiment, the Kozak sequence comprises or consists of a nucleotide sequence of SEQ ID NO: 77.

The present invention further provides a vector comprising the CAR-encoding nucleic acid. In one aspect, the CAR-encoding nucleic acid or CAR vector may be directly transduced into a cell, e.g., a T cell such as a T lymphocyte. In one aspect, the vector is a cloning or expression vector, for example including, but not limited to, one or more plasmids (e.g., expression plasmids, cloning vectors, mini circles, microcarriers, double mini chromosomes), retroviral and lentiviral vector constructs. In one aspect, the vector is capable of expressing the CAR molecule in a mammalian T cell. In one aspect, the mammalian T cell is a human T cell, such as a T lymphocyte.

In one embodiment, the vector comprising the CAR-encoding nucleic acid of the present invention is a DNA, RNA, plasmid, adenoviral vector, lentiviral vector or retroviral vector, preferably a lentiviral vector. In one embodiment, the vector is a lentiviral vector.

Generally, suitable vectors contain an origin of replication, a promoter sequence, a convenient restriction endonuclease site, one or more optional markers, and/or transcription and translation terminators that function in at least one organism. In one embodiment, the vector further comprises a promoter. In one embodiment, the promoter is an EF-1$\alpha$ promoter, e.g., comprising a sequence of SEQ ID NO: 89.

To assess expression of the CAR polypeptide or the portion thereof, the expression vector introduced into cells may also contain an optional marker gene or reporter gene, or both, to facilitate identification and selection of expression cells from a population of cells transfected or infected by the viral vector. In some embodiments, the reporter gene encodes EGFP. In some embodiments, EGFP is linked to a self-cleaving peptide, such as T2A. In some embodiments, EGFP comprises or consists of an amino acid sequence set forth in SEQ ID NO: 84 or an amino acid sequence having at least 90%, 95%, 96%, 97%, 98% or 99% identity thereto. In some embodiments, T2A comprises or consists of an amino acid sequence of SEQ ID NO: 83.

Methods for introducing and expressing genes into cells are known in the art. In the context of expression vectors, the vector may be readily introduced into a host cell such as a mammalian, bacterial, yeast or insect cell by any method known in the art. For example, the expression vector may be transferred into a host cell by physical, chemical or biological means.

Physical methods for introducing polynucleotides into a host cell include calcium phosphate precipitation, lipofection, particle bombardment, microinjection, electroporation, and the like. Methods for producing cells comprising vectors and/or exogenous nucleic acids are well known in the art. One preferred method for introducing polynucleotides into a host cell is lipofection.

## IV. Cells and drugs

In another aspect, the present invention relates to a cell comprising the vector or nucleic acid. In one embodiment, the cell is a human T cell, e.g., the T cell described herein. In one embodiment, the T cell is a T lymphocyte. In one embodiment, the cell is an autologous T cell. In one embodiment, the cell is an allogeneic T cell.

Prior to expansion and genetic modification, cells (e.g., T cells) are obtained from a subject. The term "subject" is meant to include living organisms (e.g., mammals) in which an immune response may be elicited. Examples of the subject include domestic animals (e.g., cattle, sheep, cats, dogs, and horses), primates (e.g., human and non-human primates such as monkeys), rabbits, and rodents (e.g., mice and rats). In some embodiments, the individual or subject is a human. T cells may be obtained from a number of sources, including peripheral blood mononuclear cells, bone marrow, lymph node tissues, cord blood, thymus tissues, tissues from the site of infection, ascites, pleural effusion, spleen tissues, and tumors. In certain aspects of the present invention, a number of T cell lines available in the art may be used. In certain aspects of the present invention, T cells may be obtained from blood units collected from a subject using any of a variety of techniques (such as ficoll ™ separation) known to those skilled in the art. In a preferred aspect, the cells are obtained from the circulating blood of an individual by apheresis.

In one aspect, T cells are separated from peripheral blood lymphocytes by lysing the red blood cells and elutriating depleted monocytes, for example, by centrifugation through a PERCOLLTM gradient or a counter current centrifuge. Specific sub-populations of T cells, such as CD3+, CD28+, CD4+, CD8+, CD45RA+, and CD45RO+ T cells, may be further isolated by positive or negative selection techniques. For example, in one aspect, T cells are isolated by using an anti-CD3+ antibody and an anti-CD28+ antibody.

In another aspect, the present invention relates to a method for producing a cell, comprising transducing to the cell described herein (e.g., a T cell) a vector comprising a nucleic acid encoding a CAR molecule (e.g., the CAR molecule described herein). In one embodiment, the vector is the lentiviral vector described herein.

In another aspect, the present invention also relates to a drug comprising the CAR molecule, the nucleic acid encoding same, the vector comprising same, or the cell (e.g., a T cell) comprising same described herein.

## V. Use and method

In one aspect, the present invention provides a method for preventing or treating a tumor (e.g., cancer) or providing anti-tumor immunity in a subject, comprising administering to the subject an effective amount of a cell comprising a CAR molecule, e.g., the CAR molecule-expressing cell described herein. In one embodiment, the cell is an autologous T cell. In one embodiment, the cell is an allogeneic T cell. In one embodiment, the subject is a human. In one embodiment, the T cell is a T lymphocyte.

In some embodiments, the tumor (e.g., cancer) patient has CLDN18.2 (e.g., at an elevated level, e.g., at a nucleic acid or protein level).

In some embodiments, the tumor, e.g., cancer, includes a solid tumor and a hematological tumor as well as a metastatic lesion. In one embodiment, examples of the solid tumor include a malignant tumor. The cancer may be at an early, intermediate or advanced stage, or may be a metastatic cancer.

In some embodiments, the tumor treatment will benefit from inhibition of CLDN18.2 at a nucleic acid or protein level.

In a specific embodiment, the cell comprising a CAR molecule of the present invention is capable of inhibiting proliferation of tumor cells, e.g., tumor cells expressing CLDN18.2, such as gastric cancer cells or pancreatic cancer cells.

In some embodiments, the tumor is a tumor immune escape.

In some embodiments, the tumor is cancer, e.g., gastric cancer or pancreatic cancer.

The subject may be a mammal, e.g., a primate, preferably a higher primate, e.g., a human (e.g., an individual suffering from or at risk of suffering from the diseases described herein). In one embodiment, the subject suffers from or is at risk of suffering from the diseases described herein (e.g., cancer). In certain embodiments, the subject is receiving or has received other therapies, e.g., chemotherapy and/or radiotherapy. In some embodiments, the subject has previously received or is receiving immunotherapy.

In other aspects, the present invention provides use of a CAR molecule, a nucleic acid encoding same, a vector comprising same, or a cell comprising same in the manufacture or preparation of a drug for the use described herein, e.g., for the prevention or treatment of the related diseases or disorders mentioned herein.

In some embodiments, the CAR molecule, the nucleic acid encoding same, the vector comprising same, or the cell comprising same of the present invention delays the onset of a disorder and/or symptoms associated with the disorder.

In some embodiments, the CAR molecule, the nucleic acid encoding same, the vector comprising same, or the cell comprising same of the present invention may also be administered in combination with one or more other therapies, such as a therapeutic modality and/or an additional therapeutic agent, for the use described herein, such as for use in preventing and/or treating the related diseases or disorders mentioned herein.

In some embodiments, the therapeutic modality or therapeutic agent enhances the activity or adaptability of cells expressing a CAR molecule, or alleviates the side effect associated with administration of the cell expressing the CAR molecule, or treats a disease associated with CLDN18.2.

In some embodiments, the therapeutic modality includes surgery, radiotherapy, partial irradiation, focused irradiation, or the like. In some embodiments, the therapeutic agent is selected from a chemotherapeutic agent, a cytokine, a cytotoxic agent, a vaccine, an additional antibody, a small molecule drug, and an immunomodulatory agent. Exemplary immunomodulatory agents include an immunosuppressant or an anti-inflammatory agent.

**Examples**

Table A: SEQ ID NO numbering for sequences of exemplary antibodies and scFvs thereof of the present invention (HCDR1 defined by the AbM rules, and HCDR2, HCDR3, and LCDR1, LCDR2 and LCDR3 defined by the Kabat rules)

|  | Hz38F8 | Hz46F6 | Hz14A5 | Hz25C7 | Hz69H9 |
|---|---|---|---|---|---|
| HCDR1 | 1 | 13 | 21 | 29 | 38 |
| HCDR2 | 2 | 14 | 22/88(scFv) | 30 | 39 |
| HCDR3 | 3 | 15 | 23 | 31 | 40 |
| VH | 4 | 16 | 24/86(scFv) | 32 | 41 |
| HC | 5 | 5 | 5 | 5 | 5 |
| LCDR1 | 6 | 6 | 6 | 33/89(scFv) | 42 |
| LCDR2 | 7 | 17 | 25 | 34 | 17 |
| LCDR3 | 8 | 18 | 26 | 35 | 43 |
| VL | 9 | 19 | 27 | 36/87(scFv) | 44 |
| LC | 10 | 10 | 10 | 10 | 10 |
| scFv | 11 | 20 | 28 | 37 | 45 |
| Linker | 12 | 12 | 12 | 12 | 12 |

(continued)

|  | Hz59C1 | Hz3G3 | Hz51H10 | HE37A6 |  |
|---|---|---|---|---|---|
| HCDR1 | 46 | 54 | 61 | 68 |  |
| HCDR2 | 47 | 55 | 62 | 69 |  |
| HCDR3 | 48 | 56 | 63 | 70 |  |
| VH | 49 | 57 | 64 | 71 |  |
| HC | 5 | 5 | 5 | 5 |  |
| LCDR1 | 50 | 50 | 50 | 72 |  |
| LCDR2 | 17 | 17 | 17 | 73 |  |
| LCDR3 | 51 | 58 | 65 | 74 |  |
| VL | 52 | 59 | 66 | 75 |  |
| LC | 10 | 10 | 10 | 10 |  |
| scFv/VL(scFv) | 53 | 60 | 67 | 76 |  |
| Linker | 12 | 12 | 12 | 12 |  |

**Example 1. Construction of CAR lentiviral vectors**

CAR lentiviral plasmids were constructed based on pWPT-GFP lenti-vector (Biovector).

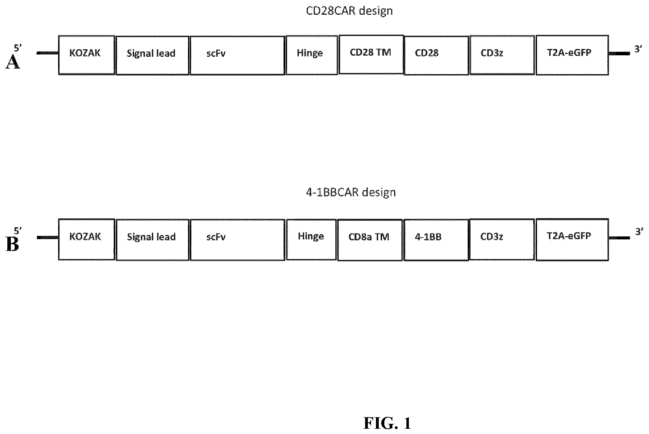

The CAR viral plasmid with an intracellular signaling region of CD28 mainly comprises the following parts (from 5 'to 3') (FIG. 1A): a KOZAK sequence, a CD8 signal peptide, a single-chain antibody scFv against Claudin18.2 (extracellular binding region), a CD8 hinge region, a CD28 transmembrane region, a CD28 co-stimulatory domain, a CD3$\zeta$ signaling domain, T2A, and EGFP.

The CAR viral plasmid with an intracellular signaling region of 4-1BB mainly comprises the following parts (from 5 'to 3') (FIG. 1B): a KOZAK sequence, a CD8 signal peptide, a single-chain antibody scFv against Claudin18.2 (extracellular binding region), a CD8 hinge region, a CD8$\alpha$ transmembrane region, a 4-1BB co-stimulatory domain, a CD3$\zeta$ signaling domain, T2A, and EGFP.

The numbering of each CAR molecule and the corresponding antibody from which the scFv is derived are shown in the following figure:

| Version | CAR molecule [a] | Anti-Claudin18.2 antibody from which the scFv is derived [b] |
|---|---|---|
| CAR with an intracellular signaling region of CD28 | 619-2-28 | HB37A6 |
|  | 652-7-28 | hz 14 A5 |
|  | 652-8-28 | hz25C7 |
|  | 723-1-28 | hz3G3 |
|  | 723-2-28 | hz46F6 |
|  | 723-3-28 | hz69H9 |
|  | 723-4-28 | hz51H10 |
|  | 723-5-28 | hz59C1 |
|  | 723-6-28 | hz38F8 |
| CAR with an intracellular signaling region of 4-1BB | 723-2-BB | hz46F6 |
|  | 723-6-BB | hz38F8 |
| [a]The controls used in the experiments were: 619-1, from patent US20170204177A1; [b]Each antibody is derived from CN 202010570517.X. | | |

See the attached sequence listing for specific sequences of the individual parts.

**Example 2. Lentiviral packaging and titer determination**

**2.1 Preparation of 293T cell line**

One day before transfection, 293T cells (ATCC: CRL-3216) in a logarithmic growth phase were digested by 0.25% trypsin, and inoculated into a T75 flask with $5 \times 10^6$ cells per dish, and DMEM high-sugar medium (Gibco, 11965-092) + 10% FBS (Hyclone, SH30406.05) + 1% penicillin + streptomycin (Gibco, 15070-063) was added to make a volume of 10 mL. The cells were cultured overnight at 37 °C with 5% $CO_2$, and were ready for packaging lentivirus when the density reached 70-80%;

**2.2 Lentiviral packaging**

The medium in the T75 flask was removed 1 h before transfection, and 6 mL of DMEM medium containing 2% FBS and 1% penicillin + streptomycin was added. The cells were placed back into an incubator at 37 °C with 5% $CO_2$;
Preparation of transfection complexes. The packaging vector and the target vector (Synbio Technologies, Suzhou) were added to 250 $\mu$L of Opti-MEM (Gibco, 31985-070) in a tube according to the amounts shown in Table 1, and mixed gently and uniformly; 28 $\mu$L of PEIpro (Polyplus, 115-010) was added to 250 $\mu$L of Opti-MEM in another tube, and mixed gently and uniformly; the PEIpro solution was added slowly to a plasmid tube, mixed gently and uniformly and left to stand for 15 min at room temperature.

Table 1 Transfection complex system

| Table 1 Component of the transfection complex system | Amount of addition |
| --- | --- |
| pWPT-CAR (CAR plasmid constructed in Example 1) | 8 ug |
| psPAX2 (Lentiviral packaging vector) | 4 ug |
| pMD2G (Lentiviral envelope vector) | 2 ug |

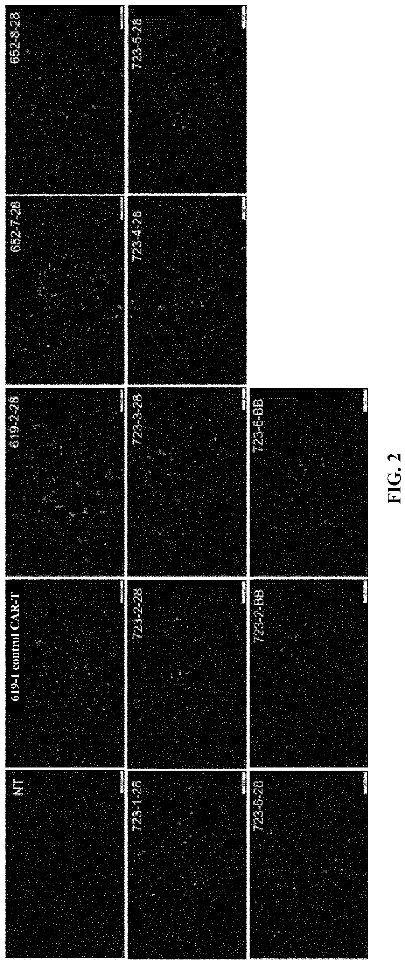

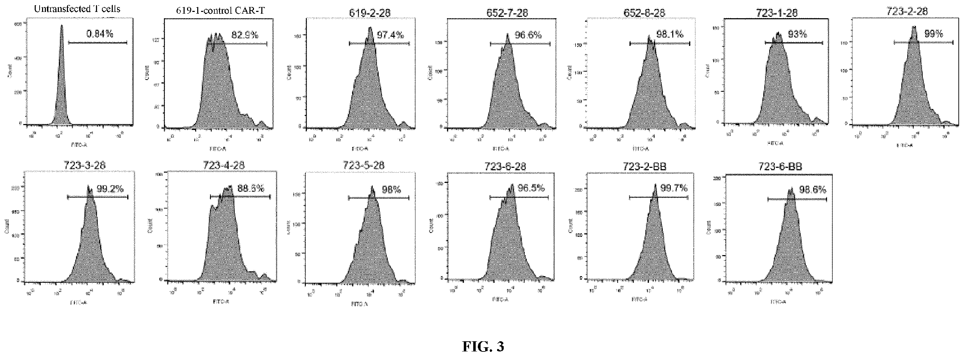

500 $\mu$L of the prepared transfection mixture system was added to a 293T cell culture flask, which was slightly shaken back and forth to uniformly distribute the liquid at the bottom of the culture flask, and cultured in an incubator at 37 °C with 5% $CO_2$; after 4 hours of transfection, the medium was discarded, and 12 mL of fresh DMEM medium containing 2% FBS and 1% penicillin + streptomycin (Gibco) was added, and cultured in an incubator with 5% $CO_2$ and at 37 °C for 48 hours.
After 48 h of transfection, cell culture supernatant was collected, stored in a refrigerator at 4 °C, and supplemented with 12 mL of fresh DMEM medium containing 2% FBS + 1% penicillin + streptomycin; GFP-positive 293T cells were detected by imaging with an immunofluorescence microscope (OLYMPUS, IX53) (FIG. 2). After 48 h of transfection, over 90% of 293T cells were GFP-positive, indicating that most 293T cells had been successfully transfected. Cell culture supernatant was collected once more 72 h after transfection and mixed with the supernatant collected after 48 h.
After 72 h of transfection, the proportion of GFP-positive 293T cells after 72 h of lentiviral packaging was measured by a flow cytometer (BD, FACSCELESTA), i.e., the packaging efficiency was verified. The results were shown in FIG. 3, wherein untransfected cells were GFP-negative, and 293T cells transfected with CARs all had a GFP-positive rate of greater than 80%, indicating that more than 80% of the cells had been successfully transfected to express GFP protein.

**2.3 Lentiviral concentration**

The cell supernatant collected in 2.2 were centrifuged at 4 °C, 2000 g for 10 min, and the supernatant was filtered into a centrifuge tube through a 0.45 $\mu$m low-protein binding filter membrane (Sartorius) to remove dead cell debris to obtain virus supernatant; the virus was concentrated using a Lenti-X Concentrator (TaKaRa, 631231) according to the manufacturer's instructions: the virus was concentrated at a ratio of virus supernatant: Lenti-X Concentrator = 3:1, mixed uniformly upside down, then placed in a refrigerator at 4 °C, and incubated overnight; after overnight incubation, the mixed solution was centrifuged at 4 °C, 1500 g for 45 min; the supernatant was carefully discarded, and the precipitate was gently suspended with 1 mL of T cell medium (RPMI 1640 medium +10% FBS +1% penicillin + streptomycin), without pipetting. The suspension was left to stand at 4 °C for 2-4 h to dissolve the virus, which was then dispensed at 0.5 mL per tube after dissolution and stored at -80 °C.

**2.4 Lentiviral titer determination**

The p24 protein content was measured by ELISA using a Lenti-X p24 Rapid Titer Kit (TaKaRa, 632200) according to the manufacturer's instructions, thereby determining the lentiviral titer rapidly.

Standards were prepared using the kit: 0 pg/mL, 12.5 pg/mL, 25 pg/mL, 50 pg/mL, 100 pg/mL, and 200 pg/mL. Samples were diluted to an appropriate fold according to a standard range.

20 $\mu$L of lysis buffer solution was added into each well of an 8-well plate according to the instructions of the kit; 200 $\mu$L of standard curve diluent, sample and the T cell medium control well were added to a detection plate precoated with a p24 antibody, and incubated at 37 °C for 60 minutes, and the plate was washed 6 times with 350 $\mu$L of washing buffer per well; 100 $\mu$L of anti-p24 (Biotin conjugate) detector antibodies was added to the wells, and incubated at 37 °C for 60 minutes, and the microtiter plate was washed; 100 $\mu$L of Streptavidin-HRP conjugate was added to each well, and incubated for 30 minutes at room temperature, and the microtiter plate was washed; 100 $\mu$L of substrate solution was added to each well immediately, and incubated for 20 minutes at room temperature in the dark; 100 $\mu$L of stop solution was added to each well to terminate the reaction; absorbance values at 450 nm and 620 nm were read using a microplate reader (Molecular Devices, SpectraMax i3) immediately after addition of the stop solution, and the virus titer was calculated according to the kit instructions (Table 2). The lentiviral titers of CARs were all higher than $10^9$ LPS/mL and were all superior to the control.

Table 2 Lentiviral titers

| Sample | 619-1 (control lentivirus) | 619-2 -28 | 652-7 -28 | 652-8 -28 | 723-1 -28 | 723-2 -28 | 723-3 -28 | 723-4 -28 | 723-5 -28 | 723-6 -28 | 723-2-BB | 723-6-BB |
|---|---|---|---|---|---|---|---|---|---|---|---|---|
| Titer ($\times 10^{10}$ LP/mL) | 1.16 | 2.02 | 2.41 | 2.93 | 2.01 | 3.24 | 2.23 | 1.53 | 2.47 | 1.60 | 3.05 | 2.43 |

**Example 3. Preparation of CAR-T cells**

A 24-well plate (Costar, 3738) was coated with 1 μg/mL recombinant humanized anti-CD3 mAb antibodies (1 μg/mL) (CytoCares, GMP-A018) and recombinant humanized anti-CD28 mAb antibodies (CytoCares, GMP-A063), which were prepared with water for injection, and coated overnight at 4 °C.

Human peripheral blood mononuclear cells (PBMCs, Allcells) were resuscitated on day 0, and cultured and activated in the coated 24-well plate. Lentiviral infection was performed on day 2, and the resuscitated PBMC cells were resuspended in complete medium RPMI-1640 (Hyclone, SH30809.01) + 10% fetal bovine serum (FBS, Hyclone, SH30084.03). Cells were added to a 24-well plate (Costar, 3524) at $3 \times 10^5$ cells/well, and 500 μL of each lentivirus prepared in Example 2 was slowly added dropwise to the cellular fluid, with the uninfected PBMC as a blank control. After 48 h of infection, the medium was changed to RPMI-1640 (Hyclone, SH30809.01) + 10% fetal bovine serum (FBS, Hyclone, SH30084.03) + 7.5 ng/mL recombinant human IL-2 (CytoCares, GMP-CD66) complete medium, and the cells were expanded to a T75 flask to be cultured for 7-9 days.

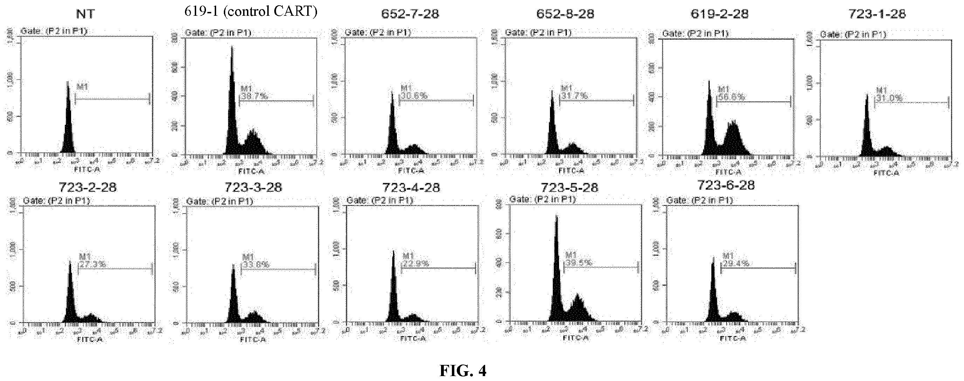

The virus-infected T cells cultured up to day 7 were centrifuged at 400 g/min for 5 min, the supernatant was removed to collect the cells. The cells were resuspended in a PBS solution containing 1% FBS, and the proportion of GFP-expressing T cells (CAR-T positive rate) was measured by a BD C6 Plus Flow Cytometer (BD, ACCURI C6 Plus). GFP + cells were CAR-positive T cells. As shown in FIG. 4, the positive rates of 9 CAR-T ranged from 25% to 60% after 7 days of infection, and were comparable or superior to that of the control CAR-T.

**Example 4. *In vitro* killing experiment of ClaudinI8.2 CAR-T on NUGC-4 tumor cells**

The CAR-T cells collected in Example 3 were resuspended in complete medium RPMI-1640 (Hyclone, SH30809.01) + 10% FBS, (Hyclone, SH30084.03), and the CAR-T cells were adjusted to $3 \times 10^5$ cells/mL according to the CAR-T cell positive rates detected in Example 3. The cells were diluted in a 3:1 gradient with the CAR-T cell density of $3 \times 10^5$ cells/mL as the starting concentration to obtain 4 concentrations. NUGC-4 tumor cells (JCRB cell bank) were genetically modified to overexpress luciferase, and resuspended in the complete medium to adjust the cell concentration to $1 \times 10^5$ cells/mL.

100 μL of the above NUGC-4 cell (target cell) suspension was added to a low-adsorption U-bottom 96-well plate (COSTAR, 7007), i.e., $1 \times 10^4$ target cells/well, and 100 μL of prepared CAR-T cell suspensions of different dilution concentrations, i.e., CAR-T cells:target cells = 3:1, 1:1, 1:3, 1:9, was added to the above wells, mixed gently and uniformly, and incubated in an incubator at 37 °C for 24 hours. 100 μL of supernatant was pipetted for cytokine detection, 100 μL of fluorescein substrate (Promega, G7940) was added to each well of the 96-well plate after the supernatant was pipetted, and the fluorescence value was detected by a microplate reader (Molecular Devices, SpectraMax i3).

Cytokines were detected using the Human Th1/Th2/Th17 Cytokine Kit (BD, 560484).

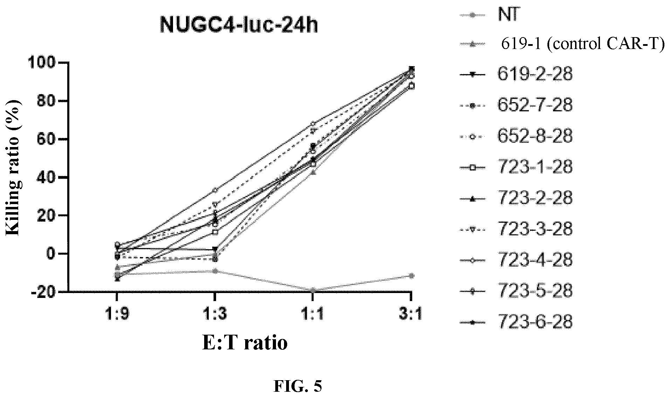

The killing ratio calculation formula is as follows: killing ratio = (NUGC-4 cell group fluorescence value - killing well fluorescence value)/NUGC-4 cell group fluorescence value. The killing efficiency of different CAR-T cells on NUGC-4 cells was shown in FIG 5, wherein 619-2-28, 652-7-28, 652-8-28, 723-1-28, 723-2-28, 723-3-28, 723-4-28, 723-5-28 and 723-6-28 all could kill target cells, and the killing efficiency was increased with the increase of the effector-to-target ratio. The killing efficiencies of 9 CAR-T were all slightly superior to that of the control CAR-T.

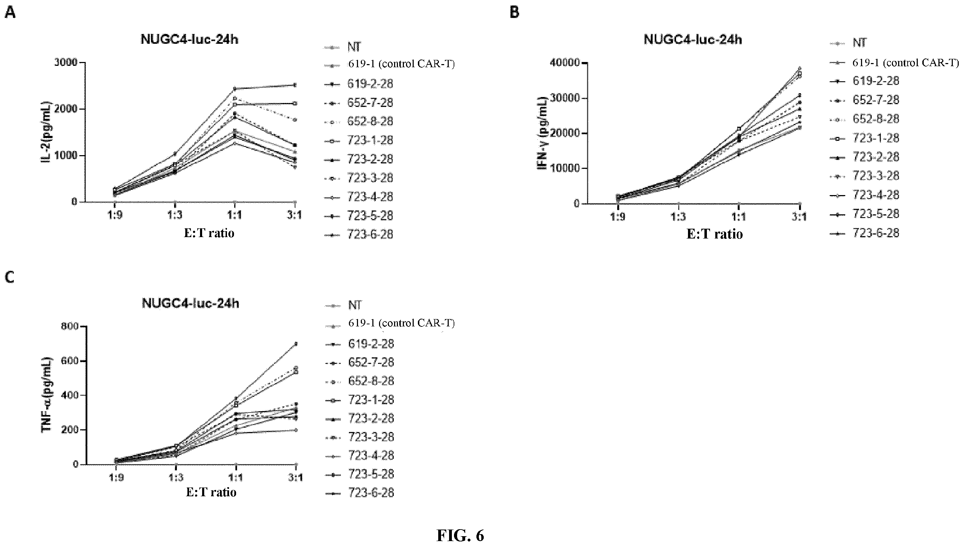

The secretion assays of IL-2 (A), INF-γ (B) and TNF-α (C) in the NUGC-4 cell killing system were shown in FIG. 6. Consistent with CAR-T killing activity, cytokine secretion levels were increased with the increase of the killing efficiency. 652-7-28, 652-8-28, 723-1-28, 723-2-28 and 723-5-28 CAR-T cells secreted higher levels of IL-2, INF-γ and TNF-α than the control CAR-T in the killing experiment.

**Example 5. *In vitro* killing experiment of ClaudinI8.2 CAR-T on DNA-G tumor cells overexpressing ClaudinI8.2**

The CAR-T cells in Example 3 were resuspended in complete medium RPMI-1640 (Hyclone, SH30809.01) + 10% fetal bovine serum (FBS, Hyclone, SH30084.03), and the CAR-T cells were adjusted to $1 \times 10^5$ cells/mL according to the positive rates of the CAR-T cells (GFP% detected by a flow cytometer) detected in Example 3. The cells were diluted in a 3:1 gradient with the CAR-T cell density of $1 \times 10^5$ cells/mL as the starting concentration to obtain 5 concentrations. DNA-G cells overexpressing luciferase and Claudin18.2 (DSMZ, as target cells, overexpressing luciferase and Claudin18.2 (UniProt ID: P56856-2)) were resuspended in the complete medium, and the cell concentration was adjusted to $1 \times 10^5$ cells/mL.

100 μL of the target cell suspension was added to a low-adsorption U-bottom 96-well plate (COSTAR, 7007), i.e., $1 \times 10^4$ target cells/well, and 100 μL of prepared CAR-T cell suspensions of different dilution concentrations, i.e., CAR-T cells:target cells = 3:1, 1:1, 1:3, 1:9, 1:27, was added to the target cell wells, mixed gently and uniformly, and incubated in an incubator at 37 °C for 24 hours. 100 μL of supernatant was pipetted for cytokine detection, and 100 μL of fluorescein substrate (Promega, G7940) was added to each well of the 96-well plate after the supernatant was pipetted, and the fluorescence value was detected by a microplate reader (Molecular Devices, SpectraMax i3).

Cytokines were detected using the Human Th1/Th2/Th17 Cytokine Kit (BD, 560484).

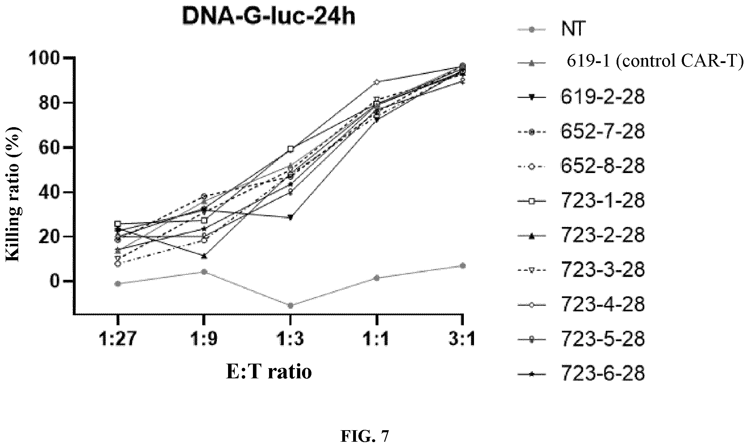

The killing ratio calculation formula is as follows: killing ratio = (DNA-G cell group fluorescence value - killing well fluorescence value)/DNA-G cell group fluorescence value. The killing efficiency of different CAR-T cells on DNA-G cells was shown in FIG. 7, wherein 9 Claudin18.2 CAR-T cells all could kill target cells specifically, and the killing efficiency was increased with the increase of the effector-to-target ratio.

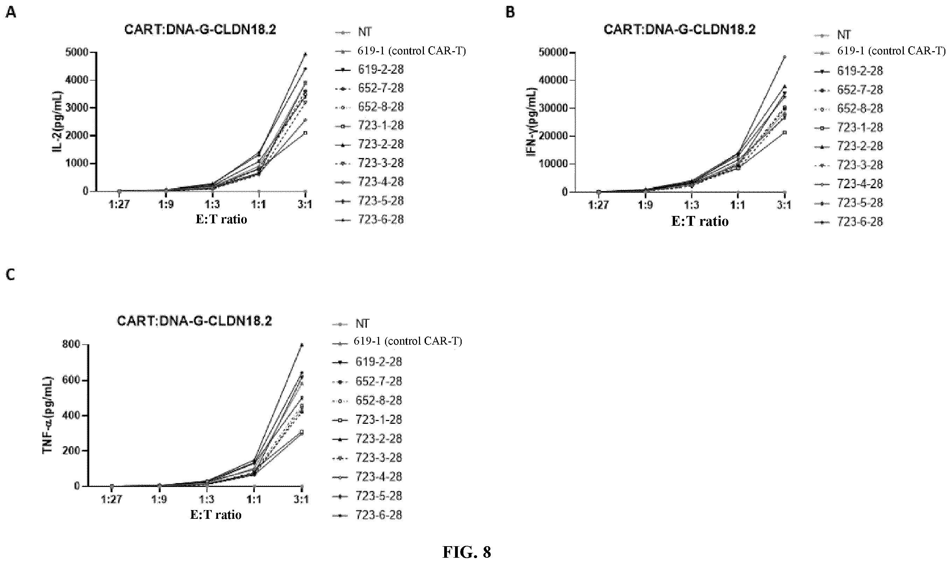

The secretion levels of IL-2 (A), INF-$\gamma$ (B) and TNF-$\alpha$ (C) in the DNA-G cell killing system were shown in FIG. 8. Consistent with CAR-T killing activity, cytokine secretion levels were increased with the increase of the killing efficiency. 619-2-28, 723-2-28 and 723-6-28 CAR-T cells secreted higher levels of IL-2, INF-$\gamma$ and TNF-$\alpha$ than the control CAR-T in the killing experiment.

**Example 6. Proliferation activity detection of Claudin18.2 CAR-T**

The CAR-T cells in Example 3 were resuspended in complete medium RPMI-1640 (Hyclone, SH30809.01) + 10% fetal bovine serum (FBS, Hyclone, SH30084.03). Far-Red reagent (Thermo Fisher, C34564) was used to label the CAR-T cells, and the CAR-T cells were adjusted to $1\times10^5$ cells/mL according to the positive rates of the CAR-T cells (GFP% detected by a flow cytometer) detected in Example 3. NUGC-4 cells (target cells, JCRB cell bank) were genetically modified to overexpress luciferase, and resuspended in the complete medium to adjust the cell concentration to $1\times10^5$ cells/mL.

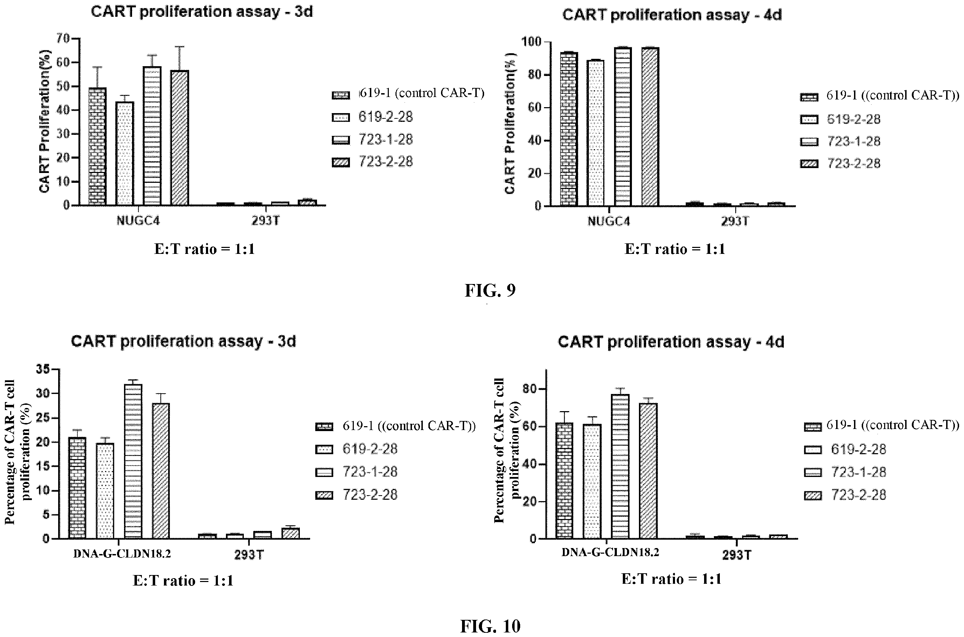

100 $\mu$L of the target cell or non-target cell (293T cell) suspension was added to a low-adsorption U-bottom 96-well plate (COSTAR, 7007), i.e., $1\times10^4$ cells/well, and 100 $\mu$L of the above CAR-T cell suspension, i.e., CAR-T cells:target cells/non-target cells = 1:1, was added to the target cell wells, mixed gently and uniformly, and incubated in an incubator at 37 °C for 72 hours and 96 hours. The 488 and APC double positive cells were detected by a flow cytometer (BD, FACSCELESTA), and the 488-positive APC-negative cells were used as proliferative cells. The proportion of the proliferative cells was calculated by FACSDiva software (BD, Celestsa). Proliferation proportions of different CAR-T cells were shown in FIG. 9. After 3 days of co-culture with tumor cells, the proliferation proportions of the CAR-T cells of the present invention exceeded 40%; after four days of co-culture, the proliferation proportions of the CAR-T cells were all greater than 80%. The proliferation proportions of the CAR-T cells co-cultured with the non-target cell 293T were all lower than 5%, indicating that Claudin18.2-positive tumor cells can promote CAR-T cell specific proliferation.

The CAR-T cells in Example 3 were resuspended in complete medium RPMI-1640 (Hyclone, SH30809.01) + 10% fetal bovine serum (FBS, Hyclone, SH30084.03). Far-Red reagent (Thermo Fisher, C34564) was used to label the CAR-T cells, and the CAR-T cells were adjusted to $1\times10^5$ cells/mL according to the positive rates of the CAR-T cells (GFP% detected by a flow cytometer) detected in Example 3. DNA-G cells overexpressing luciferase and Claudin18.2 (target cells) or 293T non-target cells were resuspended in the complete medium, and the cell concentration was adjusted to $1\times10^5$ cells/mL. 100 $\mu$L of the target cell suspension was added to a low-adsorption U-bottom 96-well plate (COSTAR, 7007), i.e., $1\times10^4$ cells/well, and 100 $\mu$L of the CAR-T cell suspension, i.e., CAR-T cells:target cells/non-target cells = 1:1, was added to the target cell wells, mixed gently and uniformly, and incubated in an incubator at 37 °C for 72 hours and 96 hours. The 488 and APC double positive cells were detected by a flow cytometer (BD, FACSCELESTA). The 488-positive APC-negative cells were used as proliferative cells. The proportion of the proliferative cells was calculated by FACSDiva software (BD, Celestsa). Proliferation proportions of different CAR-T cells were shown in FIG. 10.

After 3 days of co-culture with tumor cells, the proliferation proportions of the CAR-T cells exceeded 20%; after four days of co-culture, the proliferation proportions of the CAR-T cells were all greater than 50%. The proliferation proportions of the CAR-T cells co-cultured with the non-target cell were all lower than 5%, indicating that the Claudin18.2-positive tumor cells can promote CAR-T cell specific proliferation. After co-incubation with the target cell, the proliferation proportion of 619-2-28 CAR-T was comparable to that of the control, and the proliferation proportions of 723-1-28 and 723-2-28 CAR-T were higher than that of the control CAR-T.

**Example 7. Comparison of *in vitro* activity of CAR-T cells with intracellular signals of CD28 and 4-1BB, respectively**

**7.1 Preparation of CAR-T cells with an intracellular signaling region of 4-1BB**

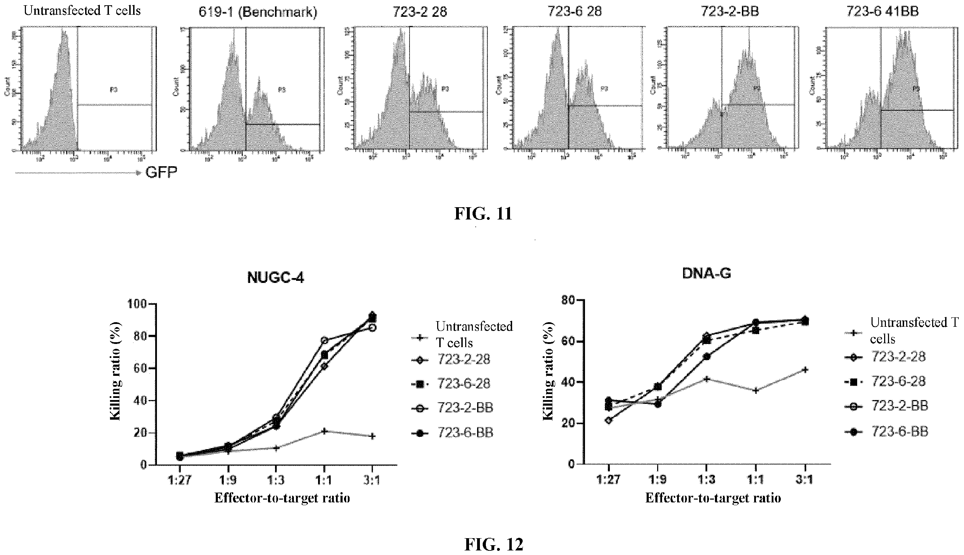

CAR-T cells with an intracellular signaling region of 4-1BB were prepared as described in Example 2. 7 days after infection, the proportion of GFP-positive T cells was detected by a flow cytometer. GFP-positive cells are CAR-positive cells. CAR-T positive rates were shown in FIG. 11 and table 3: 723-2-28 and 723-6-28 CAR-T had positive rates comparable to that of the control CAR-T, and 723-2-BB and 723-6-BB CAR-T had positive rates about twice than that of the control molecule. It indicated that the CAR with an intracellular signaling region of 4-1BB has a higher expression level.

Table 3. GPR-positive CAR-T cell proportions

| Sample | Untransfected T cells | 619-1 (control CAR-T) | 723-2-28 | 723-6-28 | 723-2-BB | 723-2-BB |
|---|---|---|---|---|---|---|
| Positive rate of CART cells | 0.20% | 36.40% | 40.50% | 41.90% | 75.60% | 72.60% |

**7.2 *In vitro* killing experiment of CAR-T with intracellular signals of CD28 and 4-1BB, respectively, on Claudin18.2-positive tumor cells**

12 days after virus infection, the CAR-T cells transfected with 723-2-28, 723-6-28, 723-2-BB and 723-6-BB prepared in Example 3 were resuspended in complete medium RPMI-1640 (Hyclone, SH30809.01) + 10% fetal bovine serum (FBS, Hyclone, SH30084.03), and the CAR-T cells were adjusted to $3 \times 10^5$ cells/mL according to the positive rates of the CAR-T cells (GFP% detected by a flow cytometer). The cells were diluted in a 3:1 gradient with the CAR-T cell density of $1 \times 10^5$ cells/mL as the starting concentration to obtain 5 concentrations.

NUGC-4 (JCRB cell bank), DNA-G tumor cells overexpressing Claudin18.2 and 293T cells were labeled with Far-Red (Invitrogen) for 10 min, washed twice and then resuspended in the complete medium with the cell concentration adjusted to $1 \times 10^5$ cells/mL. 100 $\mu$L of the above cell (target cell) suspension was added to a low-adsorption U-bottom 96-well plate (COSTAR, 7007), i.e., $1 \times 10^4$ cells/well, and 100 $\mu$L of prepared CAR-T cell suspensions of different dilution concentrations, i.e., CAR-T cells:target cells = 3:1, 1:1, 1:3, 1:9, 1:27, was added to the target cell wells, mixed gently and uniformly, and incubated in an incubator at 37 °C for 24 hours. 100$\mu$L of supernatant was pipetted for cytokine detection. The cells were centrifuged, and resuspended in propidium iodide (PI, Invitrogen) at a final concentration of 10 $\mu$g/mL. The Far-Red and PI double positive cells were detected by a flow cytometer (BD, FACSCELESTA). The killing ratio of tumor target cells was calculated by FACSDiva software (BD, Celestsa).

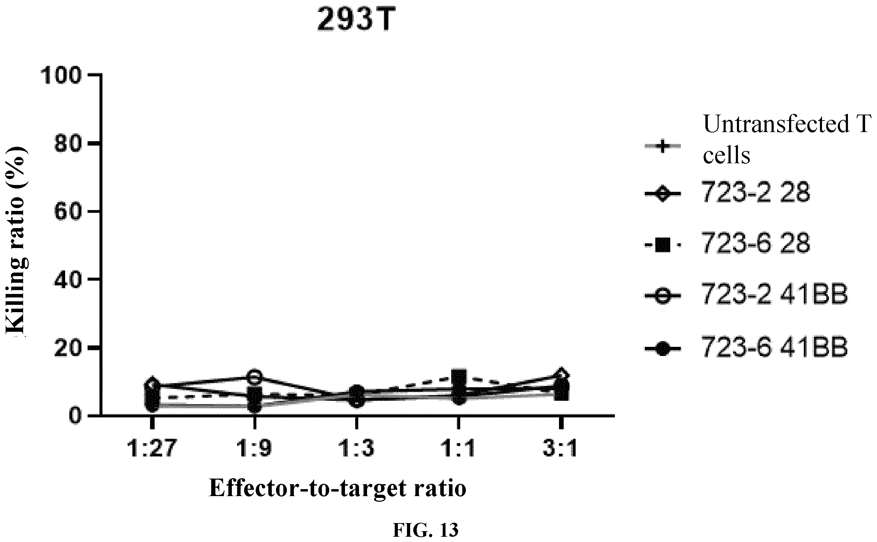

The killing efficiency of CD28 and 4-1BB versions of CAR-T cells on NUGC-4 cells was shown in FIG. 12, the CAR-T cells including 723-2-28, 723-2BB, 723-6-28 and 723-6-BB all could kill target cells, and the killing efficiency was increased with the increase of the effector-to-target ratio. The killing efficiencies of 4 CAR-T were comparable. In the Claudin18.2-negative 293T cell killing system, 723-2-28, 723-2-BB, 723-6-28, 723-6-BB did not kill non-target cells (FIG. 13).

**Example 8. Proliferation activity detection of Claudin18.2 CAR-T with intracellular signals of CD28 and 4-1BB, respectively**

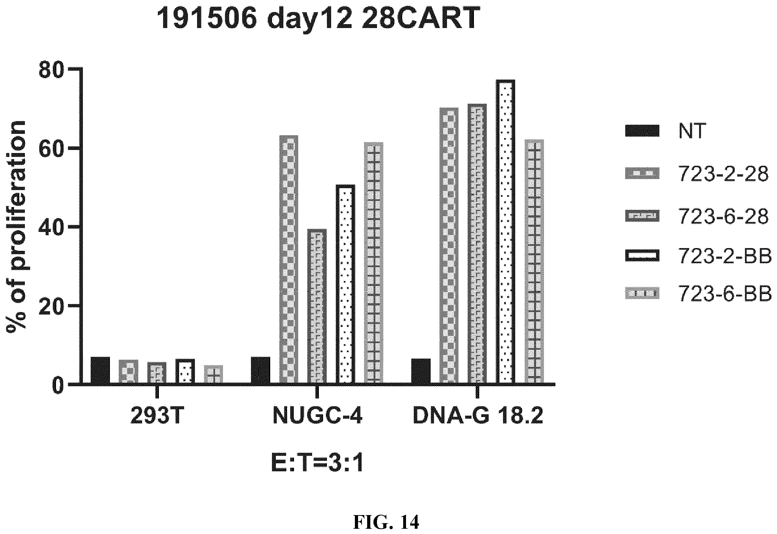

The CAR-T cells transfected with 723-2-28, 723-6-28, 723-2-BB and 723-6-BB prepared in Example 3 were resuspended in complete medium RPMI-1640 (Hyclone, SH30809.01) + 10% fetal bovine serum (FBS, Hyclone, SH30084.03). Far-Red reagent (Thermo Fisher, C34564) was used to label the CAR-T cells, and the CAR-T cells were adjusted to $3 \times 10^5$ cells/mL according to the GFP positive rates of the CAR-T cells. NUGC-4 (JCRB cell bank), DNA-G tumor cells overexpressing Claudin18.2 and 293T cells (non-target cells) were resuspended in the complete medium, and the cell concentration was adjusted to $1 \times 10^5$ cells/mL. 100 $\mu$L of the target cell suspension was added to a low-adsorption U-bottom 96-well plate (COSTAR, 7007), i.e., $1 \times 10^4$ cells/well, and 100 $\mu$L of the CAR-T cell suspension, i.e., CAR-T cells:target cells = 3:1, was added to the target cell wells, mixed gently and uniformly, and incubated in an incubator at 37 °C for 72 hours. The 488 and Far-Red double positive cells were detected by a flow cytometer (BD, FACSCELESTA). The proportion of the proliferative cells was calculated by FACSDiva software (BD, Celestsa). Proliferation proportions of different CAR-T cells were shown in FIG. 14. After 3 days of co-culture with tumor cells, the proliferation proportions of the CAR-T cells exceeded 40%; and the proliferation proportions of the CAR-T cells co-cultured with the non-target cell were all lower than 10%, indicating that the Claudin18.2-positive tumor cells can promote CAR-T cell specific proliferation. CAR-T cells with intracellular signals of CD28 and 4-1BB, respectively, were comparable in proliferation capacity.

**Example 9. Degranulation experiment to detect activation of CAR-T cells in the killing system**

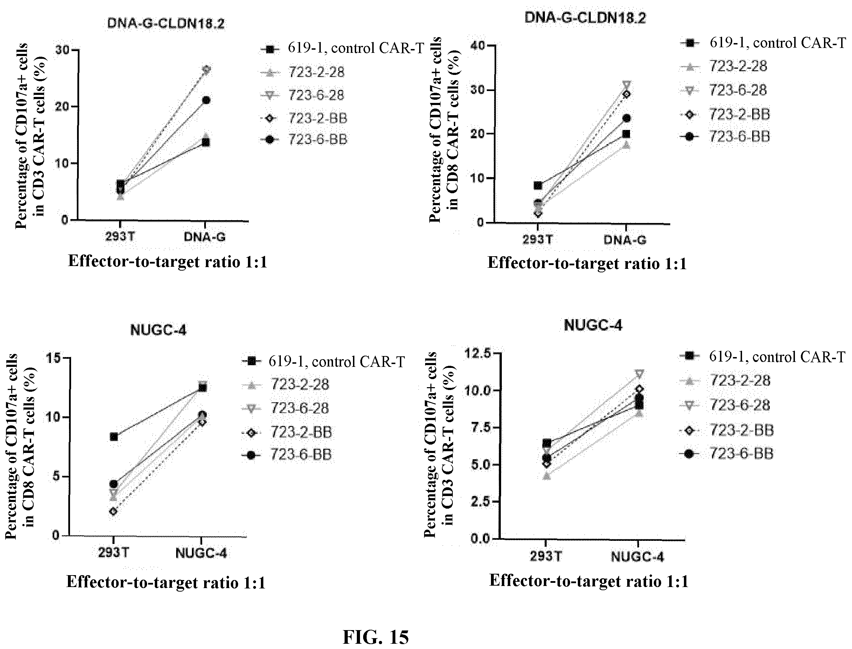

The CAR-T cells transfected with 723-2-28, 723-6-28, 723-2-BB and 723-6-BB prepared in Example 3 were co-cultured with DNA-G-CLDN18.2 cells, NUGC-4 cells and control 293T cells, respectively, at an effector-to-target ratio of 1:1. The medium contained CD107a antibodies (Biolegend, 328608). After one hour of culture, BD GolgiStop protein transport inhibitor (stabilizing monensin, BD 51-2092KZ) was added. Six hours later, after staining with CD3 antibodies (BioLegend, 317344) and CD8 antibodies (BioLegend, 344710), the expression levels of CD107a on the surfaces of CD3 and CD8 cells were detected by flow cytometry. The results were shown in FIG. 15, wherein the CD107a-positive proportions of CART cells co-incubated with 293T cells were all less than 10%. The activation proportion of CART cells incubated with DNA-G-CLDN18.2 and NUGC-4 target cells was significantly increased. Compared with the non-target cell control group, the activation amplitudes of the four CAR-T molecules after incubation with Claudi18.2-positive tumor cells were all higher than that of the control CAR-T 619-1.

## Example 10. Anti-tumor efficacy of CAR-T cells in the NUGC-4 tumor-bearing mouse model

The specific experimental steps were as follows:

NUGC-4 cells (JCRB cell bank) were routinely subcultured. NUGC-4 cells were digested by trypsin and collected by centrifugation, and resuspended in PBS. $6\times10^6$ cells were inoculated subcutaneously per NOG mouse (Vital River) into the right abdominal region. Mice were monitored for tumor volume and body weight 2 times a week.

Injection of CAR-T cells:

Tumor cells were randomly grouped 8 days after inoculation (7 mice per group), and injected in the tail vein with PBS, 619-1 CAR-T ($3\times10^6$), 723-6-28 CAR-T ($3\times10^6$), 723-6-BB CAR-T ($3\times10^6$), 723-2-28 CAR-T ($3\times10^6$), and 723-2-BB CAR-T ($3\times10^6$), respectively. Mouse tumor and body weight were measured twice weekly and monitored until day 36. Tumor volume measurement: The maximum length of the major axis (L) and the maximum length of the minor axis (W) of tumors were measured with a vernier caliper, and tumor volume was calculated by the following formula: $V = L\times W^2/2$. The mice were weighted with an electronic balance. Mice were euthanized when their body weight decreased by more than 20% throughout the study.

Statistical results and analysis:

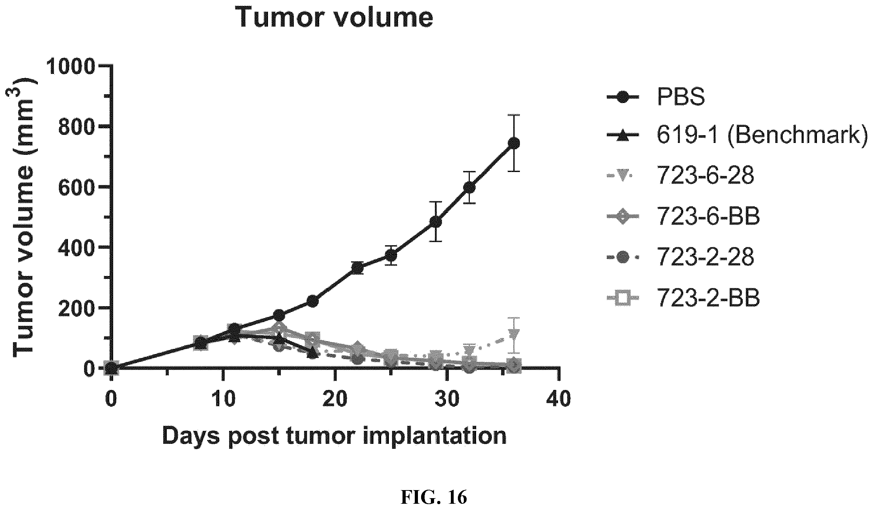

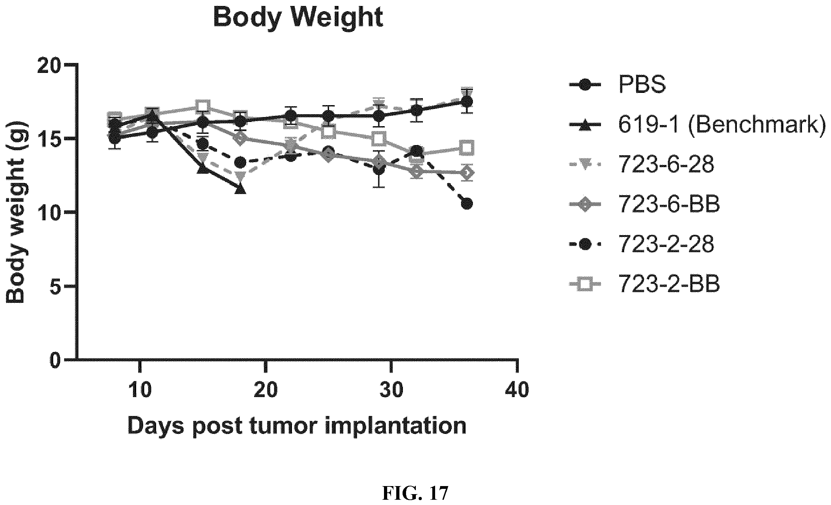

The tumor growth curves were shown in FIG. 16: the tumors in the PBS injection group continued to grow, and injection of $3\times10^6$ 723-6-28, 723-6-BB, 723-2-28, or 723-2-BB CAR-T cells inhibited the NUGC-4 tumor growth significantly (FIG. 16). Although injection of 619-1 control CAR-T could inhibit tumor growth in mice, the mice continued to lose weight after CAR-T injection and all died within three weeks, discontinuing the experiment, which showed that the control molecule was more toxic (FIG. 17). 723-6-28, 723-6-BB, 723-2-28, and 723-2-BB CAR-T could significantly inhibit tumor growth and had better safety.

## Example 11. Construction of stably expressing cell lines

### Construction of tumor cell lines overexpressing CLDN18.2

The full-length gene of humanized CLDN18.2 (UniProt ID: P56856-2) was constructed into a vector pWPT-GFP (Addgene, 12255) to replace the GFP sequence therein, and the construct was transfected, together with the packaging vectors psPAX2 (Addgene, 12260) and pMD2.G (Addgene, 12259) of the lentivirus, into HEK293T (ATCC, CRL-3216) cells for virus packaging. The culture supernatants cultured for 48 h and 72 h were each collected, and the lentivirus was concentrated using PEG8000. The pancreatic cancer DAN-G cells and gastric cancer KATO III cells were transfected with the concentrated virus, and then the cells expressing CLDN18.2 were sorted out by using a flow cytometric sorter (MoFlo XDP, Beckman Coulter) to obtain the tumor cell lines DAN-G-hCLDN18.2 and KATO III-hCLDN18.2 which were stably transfected with CLDN18.2.

## Example 12. Preparation and affinity determination of recombinant CLDN18.2 antibodies

HB37A6, hz14A5, hz25C7, hz3G3, hz46F6, hz69H9, hz51H10, hz59C1, hz38F8 (see sequence listing) and a control antibody, zolbeximab (Zmab for short, the sequence was derived from INN117), were expressed in HEK293 cells (Invitrogen, A14527) as full-length monoclonal antibodies. The expression vector was constructed firstly. Each of the paired heavy chain variable regions and light chain variable regions were placed at N-termini of the heavy chain constant region (SEQ ID NO: 5) and the light chain kappa constant region (SEQ ID NO: 10) of human IgG1, respectively, and constructed into a pcDNA3.1 expression vector with N-terminus signal peptide to obtain the light and heavy chain expression vectors. The obtained light and heavy chain expression vectors were each co-transfected with PEI (Polysciences Inc, 23966) to transiently transfect HEK293 cells, and the medium supernatant was collected after 7 days of culturing. The supernatant was purified by a Protein A column (Hitrap Mabselect Sure, GE 11-0034-95), followed by ultrafiltration and buffer-exchange into PBS (Gibco, 70011-044). The concentration was determined by the A280 method and the purity was determined by the SEC-HPLC method to obtain an antibody solution with a purity of greater than 95%.

The equilibrium dissociation constant ($K_D$) for binding of HB37A6 and the control antibody to human CLDN18.2 was determined by biolayer interferometry (BLI). A BLI affinity assay was conducted according to the existing method (Estep, P et al., High throughput solution based measurement of antibody-antigen affinity and epitope binding, MAbs, 2013.5(2): p. 270-8). The ARC (18-5060, Fortebio) sensor was soaked in SD buffer ($1\times$ PBS, BSA 0.1%, Tween-20 0.05%). 100 $\mu$L of SD buffer, each antibody and human Claudin18.2 protein (P50251802, GenScript) were added to a 96-well black polystyrene half-area microplate (Greiner, 675076), respectively. The detection was performed using Fortebio Octet Red96, and the $K_D$ values were analyzed using Fortebio Octet analysis software. The affinity of HB37A6 was 3.26E-10 which was superior to 2.14E-09 for the control antibody Zmab.

The equilibrium dissociation constant ($K_D$) for binding of the 8 humanized antibodies (Hz3G3, Hz14A5, Hz25C7, Hz38F8, Hz69H9, Hz46F6, Hz59C1 and Hz51H10) and 1 fully human antibody (HB37A6) to human CLDN18.2 was determined by surface plasmon resonance (SPR). According to the manufacturer's instructions, human Claudin18.2 (GenScript, P50251802) was coupled to the surface of a CM5 chip (GE Healthcare, 29-1496-03) using the amino coupling kit (GE Healthcare, BR-1006-33), and the remaining activation sites were blocked by injection of 1 M ethanolamine after coupling. According to the manufacturer's instructions, the binding and dissociation between the antigen on the chip surface and antibodies in the mobile phase were detected by Biacore (GE Healthcare, T200) to obtain affinity and kinetic constants. The antibodies (0-100 nM) after gradient dilution flowed over the chip surface in an order from low to high concentrations, with the binding time of 180 s and the dissociation time of 600 s. Finally, the chip was regenerated using 10 mM Glycine pH 1.5 (GE Healthcare, BR-1003-54). Kinetic analysis of the data results was performed using Biacore T200 analysis software with a 1:1 binding model. As shown in Table 4, except for Hz25C7 and Hz3G3, the other antibodies were all superior to the control antibody Zmab in terms of affinity.

Table 4. Determination of affinity constant (equilibrium dissociation constant) of CLDN18.2 antibodies by SPR

| Antibodies | ka (1/Ms) | kd (1/s) | KD (M) |
|---|---|---|---|
| Zmab | 3.482E+5 | 7.894E-4 | 2.267E-9 |
| Hz69H9 | 3.869E+5 | 1.155E-4 | 2.986E-10 |
| Hz38F8 | 3.180E+5 | 9.969E-5 | 3.135E-10 |
| Hz14A5 | 4.245E+5 | 2.081E-4 | 4.901E-10 |
| HB37A6 | 4.252E+5 | 3.063E-4 | 7.203E-10 |
| Hz59C1 | 2.482E+5 | 4.330E-4 | 1.745E-9 |
| Hz51H10 | 3.246E+5 | 6.309E-4 | 1.944E-9 |
| Hz46F6 | 1.466E+5 | 1.504E-4 | 1.026E-9 |
| Hz25C7 | 1.158E+5 | 0.001139 | 9.836E-9 |
| Hz3G3 | 5.646E+4 | 7.988E-4 | 1.415E-8 |

**Example 13. Binding of CLDN18.2 antibodies to tumor cell lines**

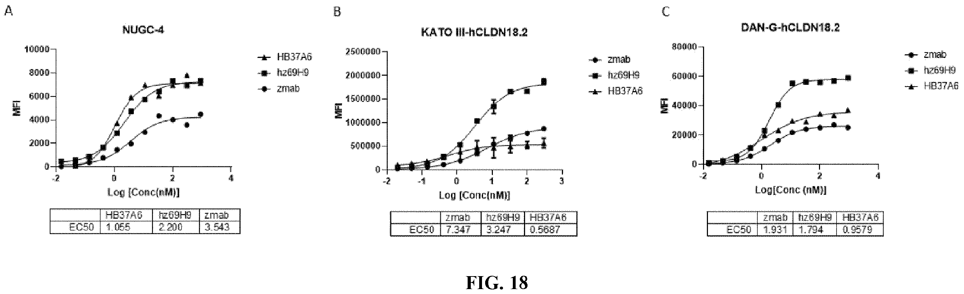

Based on the above results, 1 humanized antibody Hz69H9 and 1 fully human antibody HB37A6 were selected for further determination. The binding of the two antibodies to the gastric cancer cell line NUGC-4, the gastric cancer cell line KATO III-hCLDN18.2 and the pancreatic cancer cell line DAN-G-hCLDN18.2 which were prepared in Example 11 was determined by FACS. FIG. 18 shows that humanized antibody Hz69H9 and fully human antibody HB37A6 both have relatively good tumor cell specific binding, which is better than that of the control antibody Zmab.

**Example 14. *In vivo* anti-tumor effect of CLDN18.2 Antibodies**

**1. Activity of antibodies on the DAN-G-CLDN18.2 tumor-bearing mouse model**

The HZ69H9 and HB37A6 antibodies were selected to test their anti-tumor effect in NOD-SCID mice (60 female NOD-SCID mice (15-18 g) purchased from Beijing Vital River Laboratory Animal Technology Co., Ltd.) with human pancreatic cancer. The human pancreatic cancer cells DAN-G-hCLDN18.2 constructed in Example 11 were subcultured conventionally for subsequent *in vivo* experiments. The cells were collected by centrifugation, and the DAN-G-hCLDN18.2 cells were dispersed with PBS ($1\times$) to obtain a suspension with a cell density of $12\times10^5$ cells/mL. The cell suspension was mixed with matrigel gel at 1:1 to prepare a cell suspension with a cell concentration of $6\times10^5$ cells/mL. On day 0, 0.2 mL of the cell suspension was subcutaneously inoculated into the right abdominal region of each of the NOD-SCID mice to establish the DAN-G-CLDN18.2 tumor-bearing mouse model.

The tumor volume of each mouse was measured 5 days after tumor cell inoculation, and the mice with the tumor volume in the range of 43.36 mm$^3$-89.47 mm$^3$ were selected and divided into groups in a serpentine manner according to the size of the tumor volume (8 mice in each group).

The hIgG (Equitech-Bio, batch No. 160308-02), Hz69H9 and HB37A6, as well as the control antibody Zmab were administered, at a dose of 10 mg/kg each time, to each of the mice on days 5, 9, 12 and 16 after inoculation, and the tumor volume of the mice was monitored 2-3 times per week. Tumor volume measurement: The maximum length of the major axis (L) and the maximum length of the minor axis (W) of tumors were measured with a vernier caliper, and tumor volume was calculated by the following formula: $V = L \times W^2/2$. The mice were weighted with an electronic balance.

## 2. Activity of antibodies on the NUCG-4 tumor-bearing mouse model

The HZ69H9 and HB37A6 antibodies were selected to test their anti-tumor effect in NOG mice (100 female NOG mice (15-18 g) purchased from Beijing Vital River Laboratory Animal Technology Co., Ltd.) with human gastric cancer. The PBMC cells (Allcells) were resuscitated and centrifuged to collect cells. The PBMC cells were dispersed with PBS ($1\times$) to obtain a cell suspension with a cell density of $2.5\times10^6$ cells/mL. On day 0, 0.2 mL of cell suspension was injected into each of the NOG mice via the ophthalmic vein to establish the NOG humanized mouse model.

The NUGC-4 cells were resuscitated and subcultured conventionally for subsequent *in vivo* experiments. The cells were collected by centrifugation, and the NUGC-4 cells were dispersed with PBS ($1\times$) to obtain a suspension with a cell density of $12\times10^6$ cells/mL. The suspension was mixed with the matrigel gel at 1:1 to prepare a cell suspension with a cell concentration of $6\times10^6$ cells/mL. On day 5, 0.2 mL of the cell suspension was subcutaneously inoculated into the right abdominal region of each of the NOG humanized mice to establish the NUCG-4 tumor-bearing mouse model.

The mice were randomly divided into groups on day 1 after inoculation with tumor cells (7 mice per group). hIgG (Equitech-Bio, batch No. 160308-02), Hz69H9 and HB37A6 as well as the control antibody Zmab were administered, at a dose of 10 mg/kg each time, to each of the mice on days 1, 5, 8 and 12 after inoculation, and the tumor volume and body weight of the mice were monitored 2-3 times per week. Tumor volume measurement: The maximum length of the major axis (L) and the maximum length of the minor axis (W) of tumors were measured with a vernier caliper, and tumor volume was calculated by the following formula: $V = L \times W^2/2$. The mice were weighted with an electronic balance.

## 3. Results

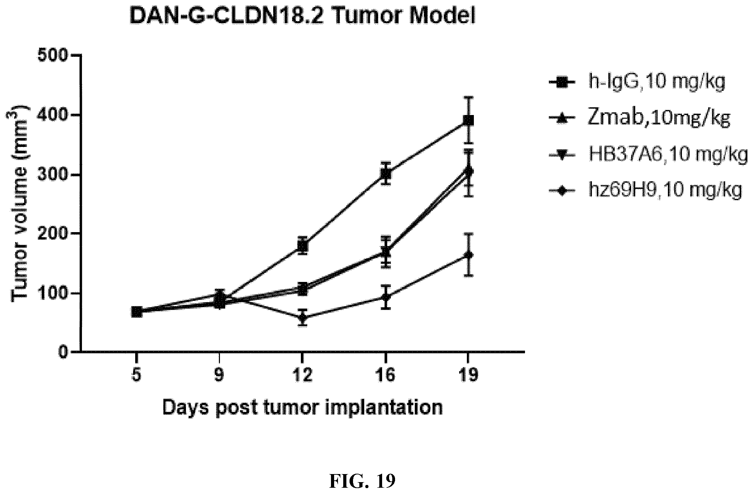

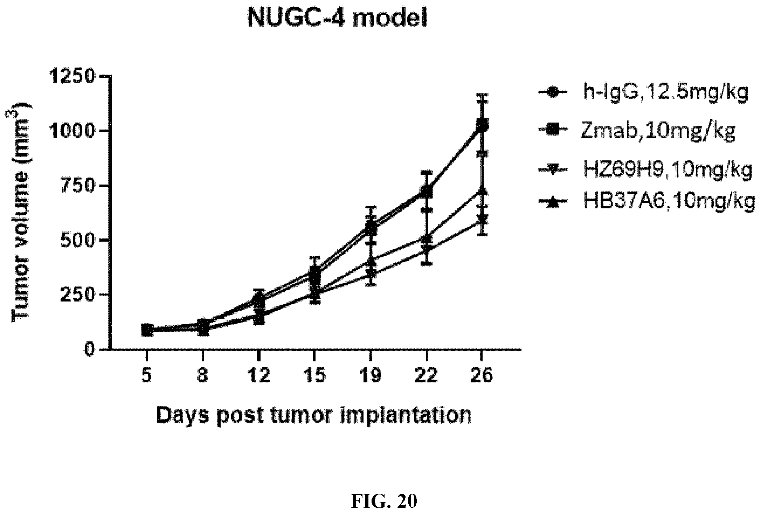

The results are shown in FIG. 19, wherein Hz69H9 and HB37A6 as well as the control antibody Zmab all can inhibit tumor growth in the human pancreatic cancer DAN-G-hCLDN18.2 mouse model. As shown in FIG. 20, Hz69H9 and HB37A6 show better anti-tumor effect than that of the control antibody Zmab in the human gastric cancer NUGC-4 mouse model.

The relative tumor growth inhibition (TGI%) was calculated on day 26 of inoculation, and the calculation formula is as follows:

$$TGI\% = 100\% \times (\text{tumor volume of the control group} - \text{tumor volume of the treatment group})/(\text{tumor volume of the control group} - \text{tumor volume of the control group before administration}).$$

In the DAN-G-CLDN18.2 tumor-bearing mouse model, the TGIs of Hz69H9 and HB37A were 70% and 28%, respectively, and the TGI of Zmab was 24%;

in the NUGC-4 tumor-bearing mouse model, the TGIs of Hz69H9 and HB37A were 46% and 31%, respectively, and the TGI of Zmab was 0%.

SEQUENCE LISTING:

| SEQ ID NO | Description of sequences | Hz38F8 |
|---|---|---|
| 1 | HCDR1 | GFSFSDYGMH |

(continued)

| SEQ ID NO | Description of sequences | Hz38F8 |
|---|---|---|
| 2 | HCDR2 | YINSGSRTLYYADTVKG |
| 3 | HCDR3 | NAYYGNAMDY |
| 4 | VH (Heavy chain variable region) | EVQLVESGGGLVQPGGSLRLSCAASGFSFSDYGMHWVRQAPGKGLE WVSYINSGSRTLYYADTVKGRFTISRDNAKNSLYLQMNSLRDEDTAV YYCARNAYYGNAMDYWGQGTLVTVSS |
| 5 | HC (Heavy chain constant region) | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KAEPKSCDKTHTCPPCPAPELLGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SRDELTKNQVSLTCLVKGFYPSDIAVEWESNGQPENNYKTTPPVLDS DGSFFLYSKLTVDKSRWQQGNVFSCSVMHEALHNHYTQKSLSLSPG K |
| 6 | LCDR1 | KSSQSLLNSGNQRNYLT |
| 7 | LCDR2 | WSSTRGS |
| 8 | LCDR3 | QNVYYYPFT |
| 9 | VL (Light chain variable region) | DIVMTQSPDSLAVSLGERATINCKSSQSLLNSGNQRNYLTWYQQKPG QPPKLLIYWSSTRGSGVPDRFSGSGSGTDFTLTISSLQAEDVAVYYCQ NVYYYPFTFGQGTRLEIK |
| 10 | LC (Light chain constant region) | RTVAAPSVFIFPPSDEQLKSGTASVVCLLNNFYPREAKVQWKVDNAL QSGNSQESVTEQDSKDSTYSLSSTLTLSKADYEKHKVYACEVTHQGL SSPVTKSFNRGEC |
| 11 | scFv | EVQLVESGGGLVQPGGSLRLSCAASGFSFSDYGMHWVRQAPGKGLE WVSYINSGSRTLYYADTVKGRFTISRDNAKNSLYLQMNSLRDEDTAV YYCARNAYYGNAMDYWGQGTLVTVSSGGGGSGGGGSGGGGSDIV MTQSPDSLAVSLGERATINCKSSQSLLNSGNQRNYLTWYQQKPGQPP KLLIYWSSTRGSGVPDRFSGSGSGTDFTLTISSLQAEDVAVYYCQNVY YYPFTFGQGTRLEIK |
| 12 | Linker | GGGGSGGGGSGGGGS |
| | | Hz46F6 |
| 13 | HCDR1 | GFSLTDYGVS |
| 14 | HCDR2 | VMWGGGNTYYNSALKS |
| 15 | HCDR3 | QRYGGNAMDY |
| 16 | VH | QVQLQESGPGLVKPSETLSLTCTVSGFSLTDYGVSWIRQPPGKGLEWI GVMWGGGNTYYNSALKSRVTISVDTSKNQFSLKLSSVTAADTAVYY CARQRYGGNAMDYWGQGTLVTVSS |
| 5 | HC | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KAEPKSCDKTHTCPPCPAPELLGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SRDELTKNQVSLTCLVKGFYPSDIAVEWESNGQPENNYKTTPPVLDS DGSFFLYSKLTVDKSRWQQGNVFSCSVMHEALHNHYTQKSLSLSPG K |

(continued)

| SEQ ID NO | Description of sequences | Hz38F8 |
|---|---|---|
| 6 | LCDR1 | KSSQSLLNSGNQRNYLT |
| 17 | LCDR2 | WASTRES |
| 18 | LCDR3 | QNSYFYPFT |
| 19 | VL | DIVMTQSPDSLAVSLGERATINCKSSQSLLNSGNQRNYLTWYQQKPG QPPKLLIYWASTRESGVPDRFSGSGSGTDFTLTISSLQAEDVAVYYCQ NSYFYPFTFGGGTKVEIK |
| 10 | LC | RTVAAPSVFIFPPSDEQLKSGTASVVCLLNNFYPREAKVQWKVDNAL QSGNSQESVTEQDSKDSTYSLSSTLTLSKADYEKHKVYACEVTHQGL SSPVTKSFNRGEC |
| 20 | scFv | QVQLQESGPGLVKPSETLSLTCTVSGFSLTDYGVSWIRQPPGKGLEWI GVMWGGGNTYYNSALKSRVTISVDTSKNQFSLKLSSVTAADTAVYY CARQRYGGNAMDYWGQGTLVTVSSGGGGSGGGGSGGGGSDIVMT QSPDSLAVSLGERATINCKSSQSLLNSGNQRNYLTWYQQKPGQPPKL LIYWASTRESGVPDRFSGSGSGTDFTLTISSLQAEDVAVYYCQNSYFY PFTFGGGTKVEIK |
| 12 | Linker | GGGGSGGGGSGGGGS |
| | | Hz14A5 |
| 21 | HCDR1 | GFSLNSYGVG |
| 22 | HCDR2 | VIWGDVSTNYHSVLIS (of monoclonal antibody) |
| 88 | HCDR2(scFv) | VIWGDGSTNYHSVLIS |
| 23 | HCDR3 | ITRGNAMDY |
| 24 | VH | QVQLQESGPGLVKPSETLSLTCTVSGFSLNSYGVGWIRQPPGKGLEW IGVIWGDVSTNYHSVLISRVTISKDTSKNQVSLKLSSVTAADTAVYYC ARITRGNAMDYWGQGTLVTVSS (of monoclonal antibody) |
| 5 | HC | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KAEPKSCDKTHTCPPCPAPELLGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SRDELTKNQVSLTCLVKGFYPSDIAVEWESNGQPENNYKTTPPVLDS DGSFFLYSKLTVDKSRWQQGNVFSCSVMHEALHNHYTQKSLSLSPG K |
| 6 | LCDR1 | KSSQSLLNSGNQRNYLT |
| 25 | LCDR2 | WASTRKS |
| 26 | LCDR3 | QNNYLFPLT |
| 27 | VL | DIVMTQSPDSLAVSLGERATINCKSSQSLLNSGNQRNYLTWYQQKPG QPPKLLIYWASTRKSGVPDRFSGSGSGTDFTLTISSLQAEDVAVYYCQ NNYLFPLTFGGGTKVEIK |
| 10 | LC | RTVAAPSVFIFPPSDEQLKSGTASVVCLLNNFYPREAKVQWKVDNAL QSGNSQESVTEQDSKDSTYSLSSTLTLSKADYEKHKVYACEVTHQGL SSPVTKSFNRGEC |

(continued)

| SEQ ID NO | Description of sequences | Hz38F8 |
|---|---|---|
| 28 | scFv | QVQLQESGPGLVKPSETLSLTCTVSGFSLNSYGVGWIRQPPGKGLEW IGVIWGDGSTNYHSVLISRVTISVDTSKNQFSLKLSSVTAADTAVYYC ARITRGNAMDYWGQGTLVTVSSGGGGSGGGGSGGGGSDIVMTQSP DSLAVSLGERATINCKSSQSLLNSGNQRNYLTWYQQKPGQPPKLLIY WASTRKSGVPDRFSGSGSGTDFTLTISSLQAEDVAVYYCQNNYLFPLT FGGGTKVEIK |
| 86 | VH (scFv) | QVQLQESGPGLVKPSETLSLTCTVSGFSLNSYGVGWIRQPPGKGLEW IGVIWGDGSTNYHSVLISRVTISVDTSKNQFSLKLSSVTAADTAVYYC ARITRGNAMDYWGQGTLVTVSS |
| 12 | Linker | GGGGSGGGGSGGGGS |
| | | Hz25C7 |
| 29 | HCDR1 | GFTFSDYGMA |
| 30 | HCDR2 | FINNLAYSIYYVDTVTG |
| 31 | HCDR3 | FTTGNVMDY |
| 32 | VH | EVQLVESGGGLVQPGGSLRLSCAASGFTFSDYGMAWVRQAPGKGLE WVAFINNLAYSIYYVDTVTGRFTISRDNAKNSLYLQMNSLRAEDTAV YYCARFTTGNVMDYWGQGTLVTVSS |
| 5 | HC | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KAEPKSCDKTHTCPPCPAPELLGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SRDELTKNQVSLTCLVKGFYPSDIAVEWESNGQPENNYKTTPPVLDS DGSFFLYSKLTVDKSRWQQGNVFSCSVMHEALHNHYTQKSLSLSPG K |
| 33 | LCDR1 | KSSQSLLQGGNQKNYLT (of monoclonal antibody) |
| 89 | LCDR1(scFv) | KSSQSLLNGGNQKNYLT |
| 34 | LCDR2 | WSSTRES |
| 35 | LCDR3 | QNSYSYPLT |
| 36 | VL | DIVMTQSPDSLAVSLGERATINCKSSQSLL**Q**GGNQKNYLTWYQQKPG QPPKLLIYWSSTRESGVPDRFSGSGSGTDFTLTISSLQAEDVAVYYCQ NSYSYPLTFGGGTKVEIK (of monoclonal antibody) |
| 10 | LC | RTVAAPSVFIFPPSDEQLKSGTASVVCLLNNFYPREAKVQWKVDNAL QSGNSQESVTEQDSKDSTYSLSSTLTLSKADYEKHKVYACEVTHQGL SSPVTKSFNRGEC |
| 37 | scFv | EVQLVESGGGLVQPGGSLRLSCAASGFTFSDYGMAWVRQAPGKGLE WVAFINNLAYSIYYVDTVTGRFTISRDNAKNSLYLQMNSLRAEDTAV |
| | | YYCARFTTGNVMDYWGQGTLVTVSSGGGGSGGGGSGGGGSDIVMT QSPDSLAVSLGERATINCKSSQSLLNGGNQKNYLTWYQQKPGQPPKL LIYWSSTRESGVPDRFSGSGSGTDFTLTISSLQAEDVAVYYCQNSYSY PLTFGGGTKVEIK |

(continued)

| SEQ ID NO | Description of sequences | Hz38F8 |
|---|---|---|
| 87 | VL(scFv) | DIVMTQSPDSLAVSLGERATINCKSSQSLLNGGNQKNYLTWYQQKPG QPPKLLIYWSSTRESGVPDRFSGSGSGTDFTLTISSLQAEDVAVYYCQ NSYSYPLTFGGGTKVEIK |
| 12 | Linker | GGGGSGGGGSGGGGS |
| | | Hz69H9 |
| 38 | HCDR1 | GYSFSSYNIH |
| 39 | HCDR2 | YIAPFQGDSRYNQKFKG |
| 40 | HCDR3 | LNRGNSLDY |
| 41 | VH | QVQLVQSGAEVKKPGSSVKVSCKASGYSFSSYNIHWVRQAPGQGLE WMGYIAPFQGDSRYNQKFKGRVTITADKSTSTAYMELSSLRSEDTAV YYCARLNRGNSLDYWGQGTLVTVSS |
| 5 | HC | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KAEPKSCDKTHTCPPCPAPELLGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SRDELTKNQVSLTCLVKGFYPSDIAVEWESNGQPENNYKTTPPVLDS DGSFFLYSKLTVDKSRWQQGNVFSCSVMHEALHNHYTQKSLSLSPG K |
| 42 | LCDR1 | KSSQSLFNSGNQRNYLT |
| 17 | LCDR2 | WASTRES |
| 43 | LCDR3 | QNNYIYFLT |
| 44 | VL | DIVMTQSPDSLAVSLGERATINCKSSQSLFNSGNQRNYLTWYQQKPG QPPKLLIYWASTRESGVPDRFSGSGSGTDFTLTISSLQAEDVAVYYCQ NNYIYPLTFGGGTKVEIK |
| 10 | LC | RTVAAPSVFIFPPSDEQLKSGTASVVCLLNNFYPREAKVQWKVDNAL QSGNSQESVTEQDSKDSTYSLSSTLTLSKADYEKHKVYACEVTHQGL SSPVTKSFNRGEC |
| 45 | scFv | QVQLVQSGAEVKKPGSSVKVSCKASGYSFSSYNIHWVRQAPGQGLE WMGYIAPFQGDSRYNQKFKGRVTITADKSTSTAYMELSSLRSEDTAV YYCARLNRGNSLDYWGQGTLVTVSSGGGGSGGGGSGGGGSDIVMT QSPDSLAVSLGERATINCKSSQSLFNSGNQRNYLTWYQQKPGQPPKL LIYWASTRESGVPDRFSGSGSGTDFTLTISSLQAEDVAVYYCQNNYIY PLTFGGGTKVEIK |
| 12 | Linker | GGGGSGGGGSGGGGS |
| | | Hz59C1 |
| 46 | HCDR1 | GYSITSGYGWN |
| 47 | HCDR2 | YIHFSGSTNYNPSLKS |
| 48 | HCDR3 | SGKGNAMDY |
| 49 | VH | QVQLQESGPGLVKPSETLSLTCTVSGYSITSGYGWNWIRQPPGKGLE WIGYIHFSGSTNYNPSLKSRVTISVDTSKNQFSLKLSSVTAADTAVYY CARSGKGNAMDYWGQGTLVTVSS |

(continued)

| SEQ ID NO | Description of sequences | Hz38F8 |
|---|---|---|
| 5 | HC | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKAEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 50 | LCDR1 | KSSQSLLNSGNQKNYLT |
| 17 | LCDR2 | WASTRES |
| 51 | LCDR3 | QNDYYFPFT |
| 52 | VL | DIVMTQSPDSLAVSLGERATINCKSSQSLLNSGNQKNYLTWYQQKPGQPPKLLIYWASTRESGVPDRFSGSGSGTDFTLTISSLQAEDVAVYYCQNDYYFPFTFGGGTKVEIK |
| 10 | LC | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| 53 | scFv | QVQLQESGPGLVKPSETLSLTCTVSGYSITSGYGWNWIRQPPGKGLEWIGYIHFSGSTNYNPSLKSRVTISVDTSKNQFSLKLSSVTAADTAVYYCARSGKGNAMDYWGQGTLVTVSSGGGGSGGGGSGGGGSDIVMTQSPDSLAVSLGERATINCKSSQSLLNSGNQKNYLTWYQQKPGQPPKLLIYWASTRESGVPDRFSGSGSGTDFTLTISSLQAEDVAVYYCQNDYYFPFTFGGGTKVEIK |
| 12 | Linker | GGGGSGGGGSGGGGS |
| | | Hz3G3 |
| 54 | HCDR1 | GYSFTTYWMH |
| 55 | HCDR2 | LIDPSDSETRLNQKFKD |
| 56 | HCDR3 | NRWLLG |
| 57 | VH | QVQLVQSGAEVKKPGASVKVSCKASGYSFTTYWMHWVRQAPGQGLEWMGLIDPSDSETRLNQKFKDRVTMTVDTSTSTVYMELSSLRSEDTAVYYCASNRWLLGWGQGTLVTVSS |
| 5 | HC | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKAEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 50 | LCDR1 | KSSQSLLNSGNQKNYLT |
| 17 | LCDR2 | WASTRES |
| 58 | LCDR3 | QNDYSYPLT |

(continued)

| SEQ ID NO | Description of sequences | Hz38F8 |
|---|---|---|
| 59 | VL | DIVMTQSPDSLAVSLGERATINCKSSQSLLNSGNQKNYLTWYQQKPG QPPKLLIYWASTRESGVPDRFSGSGSGTDFTLTISSLQAEDVAVYYCQ NDYSYPLTFGQGTKLEIK |
| 10 | LC | RTVAAPSVFIFPPSDEQLKSGTASVVCLLNNFYPREAKVQWKVDNAL QSGNSQESVTEQDSKDSTYSLSSTLTLSKADYEKHKVYACEVTHQGL SSPVTKSFNRGEC |
| 60 | scFv | QVQLVQSGAEVKKPGASVKVSCKASGYSFTTYWMHWVRQAPGQG LEWMGLIDPSDSETRLNQKFKDRVTMTVDTSTSTVYMELSSLRSEDT AVYYCASNRWLLGWGQGTLVTVSSGGGGSGGGGSGGGGSDIVMTQ SPDSLAVSLGERATINCKSSQSLLNSGNQKNYLTWYQQKPGQPPKLLI YWASTRESGVPDRFSGSGSGTDFTLTISSLQAEDVAVYYCQNDYSYPL TFGQGTKLEIK |
| 12 | Linker | GGGGSGGGGSGGGGS |
| | | Hz51H10 |
| 61 | HCDR1 | GYTFTKYIIQ |
| 62 | HCDR2 | YINPYNDDTKYNEKFKG |
| 63 | HCDR3 | TYYGNSFPN |
| 64 | VH | QVQLVQSGAEVKKPGASVKVSCKASGYTFTKYIIQWVRQAPGQRLE WMGYINPYNDDTKYNEKFKGRVTITRDTSASTAYMELSSLRSEDMA VYYCARTYYGNSFPNWGQGTLVTVSS |
| 5 | HC | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KAEPKSCDKTHTCPPCPAPELLGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SRDELTKNQVSLTCLVKGFYPSDIAVEWESNGQPENNYKTTPPVLDS DGSFFLYSKLTVDKSRWQQGNVFSCSVMHEALHNHYTQKSLSLSPG K |
| 50 | LCDR1 | KSSQSLLNSGNQKNYLT |
| 17 | LCDR2 | WASTRES |
| 65 | LCDR3 | QNDYSFPFT |
| 66 | VL | DIVMTQSPDSLAVSLGERATINCKSSQSLLNSGNQKNYLTWYQQKPG QPPKLLIYWASTRESGVPDRFSGSGSGTDFTLTISSLQAEDVAVYYCQ NDYSFPFTFGGGTKVEIK |
| 10 | LC | RTVAAPSVFIFPPSDEQLKSGTASVVCLLNNFYPREAKVQWKVDNAL QSGNSQESVTEQDSKDSTYSLSSTLTLSKADYEKHKVYACEVTHQGL SSPVTKSFNRGEC |
| 67 | scFv | QVQLVQSGAEVKKPGASVKVSCKASGYTFTKYIIQWVRQAPGQRLE WMGYINPYNDDTKYNEKFKGRVTITRDTSASTAYMELSSLRSEDMA VYYCARTYYGNSFPNWGQGTLVTVSSGGGGSGGGGSGGGGSDIVM TQSPDSLAVSLGERATINCKSSQSLLNSGNQKNYLTWYQQKPGQPPK LLIYWASTRESGVPDRFSGSGSGTDFTLTISSLQAEDVAVYYCQNDYS FPFTFGGGTKVEIK |
| 12 | Linker | GGGGSGGGGSGGGGS |

(continued)

| SEQ ID NO | Description of sequences | Hz38F8 |
|---|---|---|
| | | HB37A6 |
| 68 | HCDR1 | GFTFSSYVMS |
| 69 | HCDR2 | TISHSGGSTYYADSVKG |
| 70 | HCDR3 | DAPYYDILTGYRY |
| 71 | VH | EVQLLDSGGGLVQPGGSLRLSCAASGFTFSSYVMSWVRQAPGKGLN WVSTISHSGGSTYYADSVKGRFTISRDNSKNTLYLQMNSLRAEDTAV YYCAIDAPYYDILTGYRYWGQGTLVTVSS |
| 5 | HC | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KAEPKSCDKTHTCPPCPAPELLGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SRDELTKNQVSLTCLVKGFYPSDIAVEWESNGQPENNYKTTPPVLDS DGSFFLYSKLTVDKSRWQQGNVFSCSVMHEALHNHYTQKSLSLSPG K |
| 72 | LCDR1 | RASQSISSWLA |
| 73 | LCDR2 | KASSLES |
| 74 | LCDR3 | QQYNSYSYT |
| 75 | VL | DIQMTQSPSTLSASVGDRVTITCRASQSISSWLAWYQQKPGKAPKLLI YKASSLESGVPSRFSGSGSGTEFTLTISSLQPDDFATYYCQQYNSYSYT FGQGTKLEIK |
| 10 | LC | RTVAAPSVFIFPPSDEQLKSGTASVVCLLNNFYPREAKVQWKVDNAL QSGNSQESVTEQDSKDSTYSLSSTLTLSKADYEKHKVYACEVTHQGL SSPVTKSFNRGEC |
| 76 | scFv | EVQLLDSGGGLVQPGGSLRLSCAASGFTFSSYVMSWVRQAPGKGLN WVSTISHSGGSTYYADSVKGRFTISRDNSKNTLYLQMNSLRAEDTAV YYCAIDAPYYDILTGYRYWGQGTLVTVSSGGGGSGGGGSGGGGSDI QMTQSPSTLSASVGDRVTITCRASQSISSWLAWYQQKPGKAPKLLIY KASSLESGVPSRFSGSGSGTEFTLTISSLQPDDFATYYCQQYNSYSYTF GQGTKLEIK |
| 12 | Linker | GGGGSGGGGSGGGGS |
| | | **CD28 version sequences** |
| 77 | KOZAK sequence | GCCACCATGG |
| 78 | Amino acid sequence of CD8 signal peptide | MALPVTALLLPLALLLHAARP |
| 79 | Amino acid sequence of CD8 hinge region | TTTPAPRPPTPAPTIASQPLSLRPEACRPAAGGAVHTRGLDFACD |
| 80 | CD28 transmembrane region | FW VLV WGGVLACYSLLVTVAFIIFW V |
| 81 | Amino acid sequence of CD28 intracellular signaling region (CD28 co-stimulatory domain) | RSKRSRLLHSDYMNMTPRRPGPTRKHYQPYAPPRDFAAYRS |

(continued)

| SEQ ID NO | Description of sequences | Hz38F8 |
|---|---|---|
| 82 | Amino acid sequence of CD3ζ (CD3ζ signaling domain) | RVKFSRSADAPAYQQGQNQLYNELNLGRREEYDVLDKRRGRDPEM GGKPRRKNPQEGLYNELQKDKMAEAYSEIGMKGERRRGKGHDGLY QGLSTATKDTYDALHMQALPPR |
| 83 | Amino acid sequence of T2A | EGRGSLLTCGDVEENPGP |
| 84 | Amino acid sequence of EGFP | MVSKGEELFTGVVPILVELDGDVNGHKFSVSGEGEGDATYGKLTLKF ICTTGKLPVPWPTLVTTLTYGVQCFSRYPDHMKQHDFFKSAMPEGY VQERTIFFKDDGNYKTRAEVKFEGDTLVNRIELKGIDFKEDGNILGH KLEYNYNSHNVYIMADKQKNGIKVNFKIRHNIEDGSVQLADHYQQN TPIGDGPVLLPD |
| 85 | Amino acid sequence of T2A + EGFP | EGRGSLLTCGDVEENPGPMVSKGEELFTGVVPILVELDGDVNGHKFS VSGEGEGDATYGKLTLKFICTTGKLPVPWPTLVTTLTYGVQCFSRYP DHMKQHDFFKSAMPEGYVQERTIFFKDDGNYKTRAEVKFEGDTLVN RIELKGIDFKEDGNILGHKLEYNYNSHNVYIMADKQKNGIKVNFKIR HNIEDGSVQLADHYQQNTPIGDGPVLLPDNHYLSTQSALSKDPNEKR DHMVLLEFVTAAGITLGMDELYK |
| | | **4-1BB version protein sequences** |
| 77 | KOZAK sequence | GCCACCATGG |
| 78 | Amino acid sequence of CD8 signal peptide | MALPVTALLLPLALLLHAARP |
| 79 | Amino acid sequence of CD8 hinge region | TTTPAPRPPTPAPTIASQPLSLRPEACRPAAGGAVHTRGLDFACD |
| 86 | CD8α transmembrane region | IYIWAPLAGTCGVLLLSLVITLYC |
| 87 | Amino acid sequence of 4-1BB intracellular signaling region (4-1BB co-stimulatory domain) | KRGRKKLLYIFKQPFMRPVQTTQEEDGCSCRFPEEEEGGCEL |
| 82 | Amino acid sequence of CD3ζ (CD3ζ signaling domain) | RVKFSRSADAPAYQQGQNQLYNELNLGRREEYDVLDKRRGRDPEM GGKPRRKNPQEGLYNELQKDKMAEAYSEIGMKGERRRGKGHDGLY QGLSTATKDTYDALHMQALPPR |
| 83 | Amino acid sequence of T2A | EGRGSLLTCGDVEENPGP |
| 84 | Amino acid sequence of EGFP | MVSKGEELFTGVVPILVELDGDVNGHKFSVSGEGEGDATYGKLTLKF ICTTGKLPVPWPTLVTTLTYGVQCFSRYPDHMKQHDFFKSAMPEGY VQERTIFFKDDGNYKTRAEVKFEGDTLVNRIELKGIDFKEDGNILGH KLEYNYNSHNVYIMADKQKNGIKVNFKIRHNIEDGSVQLADHYQQN TPIGDGPVLLPD |
| 85 | Amino acid sequence of T2A + EGFP | EGRGSLLTCGDVEENPGPMVSKGEELFTGVVPILVELDGDVNGHKFS VSGEGEGDATYGKLTLKFICTTGKLPVPWPTLVTTLTYGVQCFSRYP DHMKQHDFFKSAMPEGYVQERTIFFKDDGNYKTRAEVKFEGDTLVN RIELKGIDFKEDGNILGHKLEYNYNSHNVYIMADKQKNGIKVNFKIR HNIEDGSVQLADHYQQNTPIGDGPVLLPDNHYLSTQSALSKDPNEKR DHMVLLEFVTAAGITLGMDELYK |

(continued)

| SEQ ID NO | Description of sequences | Hz38F8 |
|---|---|---|
| | | 619-1 (control) |
| 88 | Amino acid of scFv of CARSgen | QVQLQESGPGLIKPSQTLSLTCTVSGGSISSGYNWHWIRQPPGKGLE WIGYIHYTGSTNYNPALRSRVTISVDTSKNQFSLKLSSVTAADTAIYY CARIYNGNSFPYWGQGTTVTVSSGGGGSGGGGSGGGGSDIVMTQSP DSLAVSLGERATINCKSSQSLFNSGNQKNYLTWYQQKPGQPPKLLIY WASTRESGVPDRFSGSGSGTDFTLTISSLQAEDVAVYYCQNAYSFPYT FGGGTKLEIKR |
| 89 | EF-1α promoter | ACAGTGCAGGGGAAAGAATAGTAGACATAATAGCAACAGACATAC AAACTAAAGAATTACAAAAACAAATTACAAAAATTCAAAATTTTC CGATCACGAGACTAGCCTCGAGAAGCTTGATCGATGGCTCCGGTG CCCGTCAGTGGGCAGAGCGCACATCGCCCACAGTCCCCGAGAAG TTGGGGGGAGGGGTCGGCAATTGAACCGGTGCC |

## Claims

1. An isolated chimeric antigen receptor comprising: an extracellular binding region, a transmembrane region, and an intracellular signaling region comprising a co-stimulatory domain, which are sequentially linked, wherein the extracellular binding region binds to CLDN18.2 and comprises a combination of HCDR1, HCDR2, HCDR3, LCDR1, LCDR2, and LCDR3 as follows:

| | SEQ ID NO | | | | | | | | | | |
|---|---|---|---|---|---|---|---|---|---|---|---|
| HCDR1 | 1 | 13 | 21 | 21 | 29 | 29 | 38 | 46 | 54 | 61 | 68 |
| HCDR2 | 2 | 14 | 22 | 88 | 30 | 30 | 39 | 47 | 55 | 62 | 69 |
| HCDR3 | 3 | 15 | 23 | 23 | 31 | 31 | 40 | 48 | 56 | 63 | 70 |
| LCDR1 | 6 | 6 | 6 | 6 | 33 | 89 | 42 | 50 | 50 | 50 | 72 |
| LCDR2 | 7 | 17 | 25 | 25 | 34 | 34 | 17 | 17 | 17 | 17 | 73 |
| LCDR3 | 8 | 18 | 26 | 26 | 35 | 35 | 43 | 51 | 58 | 65 | 74 |

2. The chimeric antigen receptor according to claim 1, wherein the extracellular binding region comprises a VH and a VL comprising or consisting of amino acid sequences shown below, respectively:

SEQ ID NOs: 4 and 9;
SEQ ID NOs: 16 and 19;
SEQ ID NOs: 24 and 27;
SEQ ID NOs: 86 and 27;
SEQ ID NOs: 32 and 36;
SEQ ID NOs: 32 and 87;
SEQ ID NOs: 41 and 44;
SEQ ID NOs: 49 and 52;
SEQ ID NOs: 57 and 59;
SEQ ID NOs: 64 and 66; or
SEQ ID NOs: 71 and 75.

3. The chimeric antigen receptor according to claim 1 or 2, wherein the extracellular binding region binding to CLDN18.2 is an antibody or an antigen binding fragment thereof, e.g., an scFv.

4. The chimeric antigen receptor according to claim 3, wherein the scFv comprises or consists of an amino acid sequence selected from SEQ ID NOs: 11, 20, 28, 37, 45, 53, 60, 67, and 76, or an amino acid sequence having at least 90%, 91%, 92%, 93%, 94%, 95%, 96%, 97%, 98%, or 99% identity to the amino acid sequence.

5. The chimeric antigen receptor according to any one of claims 1-4, wherein the transmembrane region is a CD8 (e.g., CD8$\alpha$) or CD28 transmembrane region, preferably, the transmembrane region comprises or consists of a sequence of SEQ ID NO: 80 or 86, an amino acid sequence having at least 1, 2, or 3 modifications (e.g., substitutions) but not more than 20, 10, or 5 modifications (e.g., substitutions, e.g., conservative substitutions) relative to the amino acid sequence of SEQ ID NO: 80 or 86, or a sequence having at least 90% or 95% identity to the amino acid sequence of SEQ ID NO: 80 or 86; and optionally the transmembrane region is linked to the extracellular binding region via a hinge, and preferably, a hinge region comprises or consists of an amino acid sequence of SEQ ID NO: 79, or a sequence having at least 90%, 95%, 96%, or 97% identity to the amino acid sequence of SEQ ID NO: 79.

6. The chimeric antigen receptor according to any one of claims 1-5, wherein the intracellular signaling region comprises a CD3$\zeta$ signaling domain (e.g., comprising or consisting of a sequence set forth in SEQ ID NO: 82 or an amino acid sequence having at least 90%, 91%, 92%, 93%, 94%, 95%, 96%, 97%, 98%, or 99% identity thereto) and a co-stimulatory domain, preferably, the co-stimulatory domain is a functional signaling domain obtained from a protein selected from the group consisting of CD28 and 4-1BB (CD137), and preferably, the co-stimulatory domain comprises or consists of: an amino acid sequence having at least 1, 2 or 3 modifications (e.g., substitutions) but not more than 20, 10 or 5 modifications (e.g., substitutions, e.g., conservative substitutions) relative to an amino acid sequence of SEQ ID NO: 81 or 87, or a sequence having 90%, 91%, 92%, 93%, 94%, 95%, 96%, or 97% identity to the amino acid sequence of SEQ ID NO: 81 or 87.

7. The chimeric antigen receptor according to claim 6, wherein the co-stimulatory domain is located before the CD3$\zeta$ signaling domain.

8. The chimeric antigen receptor according to any one of claims 1-7, wherein the chimeric antigen receptor further comprises a signal peptide, e.g., a CD8 signal peptide, e.g., comprising or consisting of an amino acid sequence of SEQ ID NO: 78, and preferably, the signal peptide is located at the N-terminus of the extracellular binding region binding to CLDN18.2.

9. The chimeric antigen receptor according to any one of claims 1-8, wherein the chimeric antigen receptor further comprises a reporter gene, e.g., EGFP, e.g., comprising a sequence set forth in SEQ ID NO: 84 or an amino acid sequence having at least 90%, 91%, 92%, 93%, 94%, 95%, 96%, 97%, 98%, or 99% identity thereto, and preferably, the CAR molecule further comprises a self-cleaving peptide linked to the reporter gene, e.g., T2A, facilitating cleavage of the reporter gene from the chimeric antigen receptor (CAR) molecule, and e.g., comprising or consisting of an amino acid sequence of SEQ ID NO: 83.

10. The chimeric antigen receptor according to any one of claims 1-9, wherein the chimeric antigen receptor sequentially comprises the signal peptide, the extracellular binding region binding to CLDN18.2, the hinge region, the transmembrane region, the intracellular signaling region comprising the co-stimulatory domain and the signaling domain, and optionally the self-cleaving peptide and the reporter gene.

11. A nucleic acid encoding the chimeric antigen receptor according to any one of claims 1-10.

12. An expression vector comprising the nucleic acid according to claim 11.

13. The expression vector according to claim 12, wherein the expression vector is derived from a lentiviral plasmid, e.g., pWPT-GFP-lenti-vector.

14. A virus comprising the vector according to claim 12 or 13.

15. A T cell to which the nucleic acid according to claim 11, the expression vector according to claim 12 or 13, or the virus according to claim 14 is transduced, wherein preferably, the T cell is a T lymphocyte.

16. A T cell expressing the chimeric antigen receptor according to any one of claims 1-10 on a surface thereof, wherein preferably, the T cell is a T lymphocyte.

17. A method for preventing or treating a tumor (e.g., cancer) or providing anti-tumor immunity in a subject, comprising administering to the subject an effective amount of a cell comprising the chimeric antigen receptor according to any one of claims 1-10, or of the T cell according to claim 15 or 16.

18. The method according to claim 17, wherein a patient with the tumor (e.g., cancer) has CLDN18.2 (e.g., at an elevated level, e.g. at a nucleic acid or protein level), and preferably, the tumor is gastric or pancreatic cancer.

19. The method according to claim 17 or 18, wherein the cell is administered in combination with one or more other therapies, e.g., a therapeutic modality and/or an additional therapeutic agent, and preferably the therapeutic modality comprises a surgical treatment and/or radiotherapy; preferably the additional therapeutic agent is selected from a chemotherapeutic agent, a cytotoxic agent, a vaccine, an additional antibody, an anti-infective active agent, a small molecule drug, and an immunomodulatory agent.

20. Use of the chimeric antigen receptor according to any one of claims 1-10 in the preparation of a T cell or a drug comprising the T cell for preventing or treating a tumor (e.g., cancer) or providing anti-tumor immunity in a subject.

21. The use according to claim 20, wherein a patient with the tumor (e.g., cancer) has CLDN18.2 (e.g., at an elevated level, e.g., at a nucleic acid or protein level), and preferably, the tumor is gastric or pancreatic cancer.

22. A method for producing a T cell, comprising transducing to the T cell an expression vector comprising a nucleic acid encoding the CAR molecule according to any one of claims 1-10 or the nucleic acid according to claim 11, the expression vector according to claim 12 or 13.

CD28CAR design

4-1BBCAR design

FIG. 1

**FIG. 2**

**FIG. 3**

FIG. 4

FIG. 5

FIG. 6

**FIG. 7**

EP 4 269 436 A1

FIG. 8

FIG. 9

FIG. 10

FIG. 11

FIG. 12

# 293T

**FIG. 13**

FIG. 14

FIG. 15

**FIG. 16**

EP 4 269 436 A1

**FIG. 17**

EP 4 269 436 A1

FIG. 18

DAN-G-CLDN18.2 Tumor Model

Legend:
- h-IgG, 10 mg/kg
- Zmab, 10mg/kg
- HB37A6, 10 mg/kg
- hz69H9, 10 mg/kg

X-axis: Days post tumor implantation (5, 9, 12, 16, 19)
Y-axis: Tumor volume (mm$^3$) (0, 100, 200, 300, 400, 500)

FIG. 19

**NUGC-4 model**

Legend:
- h-IgG,12.5mg/kg
- Zmab,10mg/kg
- HZ69H9,10mg/kg
- HB37A6,10mg/kg

X-axis: Days post tumor implantation
Y-axis: Tumor volume (mm$^3$)

**FIG. 20**

EP 4 269 436 A1

## INTERNATIONAL SEARCH REPORT

| International application No. |
|---|
| **PCT/CN2021/141253** |

**A. CLASSIFICATION OF SUBJECT MATTER**

C07K 16/28(2006.01)i; A61K 39/395(2006.01)i; A61P 35/00(2006.01)i; A61P 35/02(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNTXT, VEN, NCBI, STN, PUBMED, ISI, 万方数据库, WANFANG DATABASE, CNKI: 序列检索, 荆华, 陈炳良, 李莉, claudin18.2, CLDN18.2, 嵌合抗原受体, chimeric antigen receptor, CAR, CAR-T, 抗体, antibod+

**C. DOCUMENTS CONSIDERED TO BE RELEVANT**

| Category* | Citation of document, with indication, where appropriate, of the relevant passages | Relevant to claim No. |
|---|---|---|
| PX | WO 2021254481 A1 (INNOVENT BIOLOGICS (SUZHOU) CO., LTD.) 23 December 2021 (2021-12-23) claims, and description, table A | 1-22 |
| A | WO 2020239005 A1 (SHANDONG BOAN BIOTECHNOLOGY CO., LTD.) 03 December 2020 (2020-12-03) claims 1-10 | 1-22 |
| A | CN 112111013 A (NANJING BIOHENG BIOTECH CO., LTD.) 22 December 2020 (2020-12-22) entire document | 1-22 |
| A | WO 2020139956 A1 (SPARX THERAPEUTICS INC.) 02 July 2020 (2020-07-02) entire document | 1-22 |
| A | CN 105315375 A (CARSGEN THERAPEUTICS (SHANGHAI) CO., LTD.) 10 February 2016 (2016-02-10) entire document | 1-22 |

☑ Further documents are listed in the continuation of Box C.    ☑ See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **09 March 2022** | **22 March 2022** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/141253**

**C.     DOCUMENTS CONSIDERED TO BE RELEVANT**

| Category* | Citation of document, with indication, where appropriate, of the relevant passages | Relevant to claim No. |
|---|---|---|
| A | 徐良额 等 (XU, Liang'e et al.). "CLDN18.2蛋白在恶性肿瘤治疗中的研究进展 (Advances of CLDN18.2 Protein in the Therapy of Malignant Tumors)" <br> 中国肿瘤临床 *(Chinese Journal of Clinical Oncology)*, <br> Vol. 46, No. 6, 31 December 2019 (2019-12-31), <br> pp. 311-315 | 1-22 |
| A | JIANG, Hua et al. "Claudin18.2-specific chimeric antigen receptor engineered T cells for the treatment of gastric cancer" <br> *J Natl Cancer Inst*, Vol. 111, No. 4, 31 December 2019 (2019-12-31), <br> pp. 409-418 | 1-22 |

Form PCT/ISA/210 (second sheet) (January 2015)

**INTERNATIONAL SEARCH REPORT**

| International application No. |
| --- |
| **PCT/CN2021/141253** |

| Box No. I        Nucleotide and/or amino acid sequence(s) (Continuation of item 1.c of the first sheet) |
| --- |

1.  With regard to any nucleotide and/or amino acid sequence disclosed in the international application, the international search was carried out on the basis of a sequence listing:

    a.  ☑ forming part of the international application as filed:

        ☑  in the form of an Annex C/ST.25 text file.

        ☐  on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

## INTERNATIONAL SEARCH REPORT

| International application No. |
| --- |
| **PCT/CN2021/141253** |

**Box No. II      Observations where certain claims were found unsearchable (Continuation of item 2 of first sheet)**

This international search report has not been established in respect of certain claims under Article 17(2)(a) for the following reasons:

1. ☑   Claims Nos.: **17-19**
   because they relate to subject matter not required to be searched by this Authority, namely:

   [1]   Claims 17-19 relate to a method for preventing or treating tumors (e.g., cancer) in a subject or providing anti-tumor immunity. That is, claims 17-19 relate to the subject matter defined in PCT Rule 39.1 that does not warrant a search conducted by the international searching authority: (4) a method for treatment of a human body or animal body by surgery or therapy and a diagnosis method implemented on the human body or animal body. The international search is formed on the basis of a use of an effective amount of cells comprising the chimeric antigen receptor according to any one of claims 1-10 or T cells of claim 15 or 16 in the preparation of a drug for preventing or treating tumors or providing anti-tumor immunity.

2. ☐   Claims Nos.:
   because they relate to parts of the international application that do not comply with the prescribed requirements to such an extent that no meaningful international search can be carried out, specifically:

3. ☐   Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2021/141253**

| Patent document cited in search report | | | Publication date (day/month/year) | Patent family member(s) | | | Publication date (day/month/year) |
|---|---|---|---|---|---|---|---|
| WO | 2021254481 | A1 | 23 December 2021 | None | | | |
| WO | 2020239005 | A1 | 03 December 2020 | KR | 20220006085 | A | 14 January 2022 |
| | | | | EP | 3929214 | A1 | 29 December 2021 |
| | | | | CA | 3136281 | A1 | 03 December 2020 |
| | | | | AU | 2020281380 | A1 | 07 October 2021 |
| | | | | BR | 112021023897 | A2 | 25 January 2022 |
| CN | 112111013 | A | 22 December 2020 | None | | | |
| WO | 2020139956 | A1 | 02 July 2020 | US | 2020207857 | A1 | 02 July 2020 |
| | | | | KR | 20210111792 | A | 13 September 2021 |
| | | | | CN | 113227135 | A | 06 August 2021 |
| | | | | EP | 3902824 | A1 | 03 November 2021 |
| CN | 105315375 | A | 10 February 2016 | KR | 20170023181 | A | 02 March 2017 |
| | | | | KR | 101950747 | B1 | 21 February 2019 |
| | | | | US | 2020172613 | A1 | 04 June 2020 |
| | | | | US | 11198729 | B2 | 14 December 2021 |
| | | | | US | 2017204177 | A1 | 20 July 2017 |
| | | | | US | 10377822 | B2 | 13 August 2019 |
| | | | | WO | 2016008405 | A1 | 21 January 2016 |
| | | | | JP | 2017522024 | A | 10 August 2017 |
| | | | | EP | 3170842 | A1 | 24 May 2017 |
| | | | | EP | 3170842 | A4 | 27 December 2017 |
| | | | | EP | 3170842 | B1 | 04 September 2019 |
| | | | | ES | 2746555 | T3 | 06 March 2020 |
| | | | | JP | 2019122380 | A | 25 July 2019 |
| | | | | JP | 6664528 | B2 | 13 March 2020 |
| | | | | PT | 3170842 | T | 30 September 2019 |
| | | | | DK | 3170842 | T3 | 07 October 2019 |
| | | | | PL | 3170842 | T3 | 31 January 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170204177 A1

- CN 202010570517X


**Non-patent literature cited in the description**

- **SINGH, P. ; TOOM, S. ; HUANG, Y.** Anti-CLDN18.2 antibody as new targeted therapy for advanced gastric cancer. *J Hematol Oncol,* 2017, vol. 10, 105, https://doi.org/10.1186/s13045-017-0473-4
- **O. TURECI. et al.** *Gene,* 2011, vol. 481, 83-92
- **CHOTHIA et al.** *Nature,* 1989, vol. 342, 877-883
- **AL-LAZIKANI et al.** Standard conformations for the canonical structures of immunoglobulins. *Journal of Molecular Biology,* 1997, vol. 273, 927-948
- **KABAT et al.** Sequences of Proteins of Immunological Interest. U.S. Department of Health and Human Services, National Institutes of Health, 1987

- **KABAT et al.** Sequences of Proteins of Immunological Interest. Public Health Service, National Institutes of Health, 1991
- **NEEDLEMA ; WUNSCH.** *J. Mol. Biol.,* 1970, vol. 48, 444-453
- **E. MEYERS ; W. MILLER.** *CABIOS,* 1989, vol. 4, 11-17
- **ESTEP, P et al.** High throughput solution based measurement of antibody-antigen affinity and epitope binding. *MAbs,* 2013, vol. 5 (2), 270-8